# EUROPEAN PATENT SPECIFICATION

(11) **EP 3 204 030 B1**
(45) Date of publication and mention of the grant of the patent: **27.04.2022**
(21) Application number: 15775453.2
(22) Date of filing: 07.10.2015
(51) Int. Cl.: A61K 38/17, A61K 31/00, A61K 38/16, C07K 14/005, A61K 38/21, A61K 31/522, C07K 14/705, A61K 45/06, A61K 31/513, A61K 31/7072, A61P 31/14, A61K 31/675, A61P 31/20

(54) **COMBINATION THERAPY OF HBV AND HDV INFECTION**
KOMBINATIONSTHERAPIE FÜR HBV- UND HDV-INFEKTION
TRAITEMENT COMBINÉ D'UNE INFECTION PAR LE VHB ET LE VHD

(30) Priority: 07.10.2014 EP 14187865
(43) Date of publication of application: 16.08.2017
(73) Proprietor: Myr GmbH, 30938 Burgwedel (DE)
(72) Inventor: ALEXANDROV, Alexander, 61348 Bad Homburg (DE)
(74) Representative: Carpmaels & Ransford LLP
(86) International application number: PCT/EP2015/073173
(87) International publication number: WO 2016/055534

(56) References cited:
- WO-A1-02/094866
- WO-A1-2009/092396
- WO-A1-2009/092396
- WO-A1-2009/092612
- WO-A1-2009/092612
- WO-A1-2013/159243
- DI BISCEGLIE ADRIAN M ET AL: "Prolonged Therapy of Advanced Chronic Hepatitis C with Low-Dose Peginterferon", NEW ENGLAND JOURNAL OF MEDICINE, vol. 359, no. 23, December 2008 (2008-12), pages 2429-2441, XP9516310, ISSN: 0028-4793
- VOLZ TASSILO ET AL: "The entry inhibitor Myrcludex-B efficiently blocks intrahepatic virus spreading in humanized mice previously infected with hepatitis B virus", JOURNAL OF HEPATOLOGY, ELSEVIER, AMSTERDAM, NL, vol. 58, no. 5, 13 December 2012 (2012-12-13), pages 861-867, XP028578660, ISSN: 0168-8278, DOI: 10.1016/J.JHEP.2012.12.008
- BENJAMIN HEIDRICH ET AL: "Late HDV RNA relapse after peginterferon alpha-based therapy of chronic hepatitis delta", HEPATOLOGY, vol. 60, no. 1, 1 July 2014 (2014-07-01), pages 87-97, XP55626440, ISSN: 0270-9139, DOI: 10.1002/hep.27102
- NKONGOLO SHIRIN ET AL: "Cyclosporin A inhibits hepatitis B and hepatitis D virus entry by cyclophilin-independent interference with the NTCP receptor", JOURNAL OF HEPATOLOGY, vol. 60, no. 4, 1 December 2013 (2013-12-01), pages 723-731, XP028632086, ISSN: 0168-8278, DOI: 10.1016/J.JHEP.2013.11.022
- SIMONE I STRASSER: "Drugs in Development for the Treatment of Chronic Hepatitis B", CURRENT HEPATITIS REPORTS, CURRENT SCIENCE INC, NEW YORK, vol. 11, no. 2, 23 March 2012 (2012-03-23) , pages 111-118, XP035063836, ISSN: 1541-0706, DOI: 10.1007/S11901-012-0131-9
- STEPHAN URBAN ET AL: "Strategies to Inhibit Entry of HBV and HDV Into Hepatocytes", GASTROENTEROLOGY, vol. 147, no. 1, 1 July 2014 (2014-07-01), pages 48-64, XP055175233, ISSN: 0016-5085, DOI: 10.1053/j.gastro.2014.04.030
- DI BISCEGLIE ADRIAN M ET AL: "Prolonged Therapy of Advanced Chronic Hepatitis C with Low-Dose Peginterferon", NEW ENGLAND JOURNAL OF MEDICINE, vol. 359, no. 23, December 2008 (2008-12), pages 2429-2441, XP9516310, ISSN: 0028-4793
- VOLZ TASSILO ET AL: "The entry inhibitor Myrcludex-B efficiently blocks intrahepatic virus spreading in humanized mice previously infected with hepatitis B virus", JOURNAL OF HEPATOLOGY, ELSEVIER, AMSTERDAM, NL, vol. 58, no. 5, 13 December 2012 (2012-12-13), pages 861-867, XP028578660, ISSN: 0168-8278, DOI: 10.1016/J.JHEP.2012.12.008
- BENJAMIN HEIDRICH ET AL: "Late HDV RNA relapse after peginterferon alpha-based therapy of chronic hepatitis delta", HEPATOLOGY, vol. 60, no. 1, 1 July 2014 (2014-07-01), pages 87-97, XP55626440, ISSN: 0270-9139, DOI: 10.1002/hep.27102
- COOKSLEY W G E ET AL: "PEGINTERFERON ALPHA-2A (40 KDA): AN ADVANCE IN THE TREATMENT OF HEPATITIS B E ANTIGEN-POSITIVE CHRONIC HEPATITIS B", JOURNAL OF VIRAL HEPATITIS, BLACKWELL PUBLISHING LTD, OXFORD, UK, vol. 10, no. 4, 1 January 2003 (2003-01-01), pages 298-305, XP008075233, ISSN: 1352-0504, DOI: 10.1046/J.1365-2893.2003.00450.X

## Description

### FIELD

The present disclosure pertains to novel compositions for the treatment of infections caused by hepatitis B virus (HBV) and hepatitis D virus (HDV), in particular hepatitis B and D. The compositions provided herein comprise a combination of an agent acting as an HBV/HDV entry inhibitor, and a nucleotide/nucleoside analogue (NUC) or immunomodulator such as interferon.

### BACKGROUND

### Epidemiology and medical problem related to Hepatitis B virus

Hepatitis B is a potentially life-threatening liver infection caused by the HBV. Worldwide, an estimated two billion people were HBV infected, whereas more than 350 million of those have chronic liver infections (WHO Fact Sheet, 2008).

Chronic hepatitis B (CHB) is a serious global public health issue. Up to 1.2 million deaths per year are caused by HBV-related chronic liver diseases resulting in it being the 10th leading cause of death worldwide (Lavanchy, 2004). Without appropriate treatment, 15-25% of the chronically infected will die prematurely because of the severe and fatal complications of chronic liver disease (CDC Fact Sheet, 2006). Actually, only a relatively small proportion of affected patients receive medication.

### Epidemiology and medical problem related to Hepatitis D virus

About 5% of chronically HBV infected are co-infected with HDV (Taylor, 2006). HDV is a small RNA virus, which requires helper functions from HBV for virion assembly and propagation, and uses the HBV envelope for virus release and infection of new cells. Immunization against HBV also protects against HDV infection. The presence of HDV is associated with more severe and rapid progression of liver disease than HBV infection alone. Liver cirrhosis and cancer occur 10-15 years earlier in HBV/HDV co-infection, and the 5-year mortality of co-infected individuals is twice that of HBV mono-infection (German Guideline for Prophylaxis, Diagnostics and Treatment of HBV infection, 2007).

The therapeutic options for HDV co-infected patients are very limited. Only interferons show some degree of efficacy in a small proportion of patients with approximately 25% of virological and biochemical response. Antiviral agents active against HBV do not work against HDV (Taylor, 2006).

### Treatment goals

Several national guidelines for treatment of HBV infection exist, and the European Association for the Study of the Liver (EASL) clinical practice guideline has recently been published (Journal of Hepatology, 2009). According to this guideline, the goal of therapy for hepatitis B is to improve quality of life and survival by preventing progression of the disease to cirrhosis, end-stage liver disease, HCC and death. The ideal endpoint of the therapy is sustained Hepatitis B virus s antigen (HBsAg) loss with or without seroconversion to anti-HBs. This is associated with a complete and definitive remission of the activity of CHB and an improved long-term outcome. However, this goal can be achieved only in minority of patients with available therapies. Therefore, for the Hepatitis B virus e antigen (HBeAg) positive patients, a durable HBeAg seroconversion and in HBeAg negative patients or HBeAg positive patients who are not able to achieve HBeAg seroconversion, a maintained undetectable DNA level is the next most desirable goal.

### HBV virus

Hepatitis B viruses (HBVs) are small, enveloped DNA viruses that replicate their genome via reverse transcription of a pregenomic RNA transcript in the cytoplasm of infected hepatocytes. They are classified into the family *Hepadnaviridae* and are adapted to mammals (primates and rodents) and birds, where they cause acute and persistent infections. From studies in a HBVsusseptable cell line HepaRG and systems based on primary human hepatocytes (PHH) and primary *Tupaia belangeri* hepatocytes it became clear that viral infectivity can be assigned to different subdomains in two of the three viral envelope proteins. The three HBV envelope proteins are termed L (large), M (middle) and S (small). They form a proteinaceous outer virus shell, which is embedded in an endoplasmic reticulum (ER)-derived lipid bilayer. Their coding mRNAs originate from one single open reading frame. Since their three start codons are in phase, they share the C-terminal 226 amino acids long S-domain which anchor the proteins via four putative transmembrane helices (TM domains) in the lipid bilayer. The S protein drives particle formation and accordingly serves an important function in virus assembly. The S domain has further been shown to participate in virus entry. Linked to the N terminus of the S domain, the M and L proteins bear two hydrophilic extensions, one of 55 amino acids and, depending on genotype, one of 108 (genotype D), 118 (genotypes E and G), or 119 (genotypes A, B, C, F, H) amino acids called pre-S2 (M) and pre-S1 (L), respectively. The L proteins of all hepadnaviruses contain recognition motifs for *N*-myristoyltransferase and are accordingly subjected to this modification. The lack of a classical secretion signal in L results in an initial cytoplasmic orientation of its pre-S domains. After synthesis, myristoylation, and incorporation of the TM helices into the ER membrane, the pre-S domain of L traverses the lipid bilayer, allowing parts of it to become accessible at the particle exterior. In virions the stoichiometric ratio of L, M and S proteins is about 1:1:4, while the more abundantly secreted non-infectious subviral particles (SVPs) contain almost exclusively S protein and only traces of L protein.

Crucial elements for virus attachment, specific receptor binding, or fusion have been identified within the L, M, and S proteins. Myristoylation of L was found to be mandatory for virus infectivity (6, 16). However, the requirement of the N-terminal myristic acid moiety in the inhibitory activity is to some extent variable. Replacement of myristic acid by other hydrophobic saturated and unsaturated fatty acid moieties or even by cholesterol allows the modulation of specific activities according to the relative hydrophobicity. Thus, lipophilicity *per se* and not a specific lipid structure seems to play the key role for this part of the molecule.

Successive deletions of 5 amino acids within the N-terminal pre-S1 sequence of residues 1 to 78 abrogated HBV infectivity. In contrast, most parts of the pre-S2 domain were dispensable for infection. Using HDV, these results were confirmed, indicating that both viruses share very similar entry modes. It was further found that the S domain, either as part of the HBV L protein or as the S protein, being the major constituent of the viral envelope, is required for infectivity. Two separable and distinct functions were identified within the pre-S1 infectivity determinant consisting of residues 1 to 78: one is related to the inhibitory activity of the peptide through interference with a hepatocyte-specific receptor, and the other, involving amino acids 49 to 78, is still unknown.

### Current HBV therapies

Approved drugs for treatment of chronic HBV-infections belong to two classes: antivirals, and immunomodulators such as interferons.

All HBV antivirals currently available belong to the class of nucleoside/nucleotide analogues (NUCs) and act as polymerase inhibitors. More recently approved entecavir and tenofovir are considered to be superior to other approved agents (lamivudine, adefovir and telbuvudine) because of higher potency and higher genetic barrier for resistance in treatment naive patients. All approved anti-HBV drugs show good safety profiles so far.

All of these products block virus replication and lead to a rapid decrease of plasma viral DNA. Patients may also benefit from improvements in liver histology. However, HBsAg seroconversion, the ultimate goal of HBV treatment, is achieved in only 4% of tenofovir patients after two years and is even lower for the other drugs (German Guideline for Prophylaxis, Diagnostics and Treatment of HBV infection, 2007). Furthermore, 70% of patients treated with lamivudine develop resistance against the drug after five years of treatment. For adefovir, the resistance development is slower with 18% of the patients showing resistance after four years. For entecavir this figure is 1.5% in 6 years therapy in naive patients. Nevertheless, a significant population of patients resistant to nucleoside / nucleotide analogues will accumulate over the next years, especially as patients previously treated with lamivudine develop high levels of resistance to entecavir. Reverse transcriptase inhibitors do not prevent the establishment of cccDNA (covalently closed circular DNA) in naive hepatocytes.

Interferon-alpha (INFa) is an immunomodulator with a complex and not completely understood mode of action, involving activation of the immune system to eliminate the infected hepatocytes. Several formulations are marketed with a usual dose of 5 to 10 mg units two or three times weekly. Pegylated forms with delayed release from subcutaneous depots are also available, allowing more patient friendly application only once a week. All interferon treatments are administered by subcutaneous self-injection. The major drawback of interferon treatment for chronic hepatitis B is the combination of moderate efficacy (after 48 weeks of treatment, overall HBsAg seroconversion rates of approximately 3-6%, undetectable HBV DNA in about 20%, depending on the HBV genotype: Lau et al., 2005; Fink et al., 2006) and significant side effects. These may include the cytokine syndrome, severe depression and suicide and are experienced by a substantial part of the patient population. Interferons are the only treatment option showing some therapeutic effects in HBV/HDV co-infection. However, the efficacy in this population is rather limited with only 25% showing sustained virological and biochemical response (Interview with Dr. H. Wedemeyer, Hanover Medical School, Germany).

Thus, current therapies for chronic hepatitis B rarely induce serological cure and thus long-term treatment with HBV polymerase inhibitors is usually required. There is no effective treatment for the majority of hepatitis delta patients.

### HBV/HDV entry inhibitors

A novel class of anti-HBV and HDV molecules, virus entry inhibitors, has recently been described. Its postulated mechanism of antiviral action is the highly specific, highly stable binding to Na+-taurocholate cotransporting polypeptide (NTCP), a sodium/bile acid cotransporter (also known as liver bile acid transporter (LBAT)) located at the basolateral membrane of differentiated hepatocytes. NTCP binding results in abrogation of a productive fusion of the viral and the cellular membranes and thereby prevents an infection of the cell. This unique mechanism of action allows to address two most important medical needs, namely long-term HBV eradication as well as antiviral activity against hepatitis D (or, delta) virus (HDV).

Virus entry inhibitors are derived from the N-terminal domain of the large (L) HBV surface protein, pre-S1. An example of an HBV/HDV entry inhibitor is a linear 47-amino acid chemically synthesized peptide (known under the trade name Myrcludex B) which is derived from the pre-S1 domain of the large (L) HBV surface protein, composed of naturally occurring L-amino acids and bearing an N-terminal myristoyl moiety and a C-terminal carboxamide.

### Combination therapies

Across the medical community, there is a great interest in combination therapies aiming at HBsAg loss after a limited treatment period. However, the results of combination trials using interferons together with nucleoside/nucleotides in HBV and HDV infection were so far disappointing. Thus, combining pegylated Interferon-alpha with a nucleoside analogue lamivudine did not produce an increase in efficacy (Lau et al., 2005).

Taken together, there exists an unmet need for further therapies of HBV and HDV infections and resulting diseases. In particular, such therapies should aim at HBsAg loss, with or without seroconversion to anti-HBs antibodies, a result closest to complete cure.

WO 2013/159243 relates to compositions and methods of treating or preventing HBV and/or HDV infection or a disease associated with said infection in a mammal through modulation of the production and/or function of the NTCP protein and polynucleotide, or its interaction with the viruses. Nkongolo et al., J Hepatol. 2014; 60(4):723-31, characterizes the effect of cyclosporin A on HBV/HDV infection. Strasser., Current Hepatitis Reports. 2012; 11: 111-118, relates to drugs in development for the treatment of chronic hepatitis B. Urban et al., Gastroenterology. 2014; 147(1): 48-64, relates to strategies to inhibit entry of HBV and HDV into hepatocytes. WO 2009/092612 relates to hydrophobic modified preS-derived peptides of hepatitis B virus (HBV) which are versatile vehicles for the specific delivery of compounds to the liver, preferably to hepatocytes, in vitro as well as in vivo. WO 2009/092396 relates to hydrophobic modified preS-derived peptides of hepatitis B virus (HBV) which are derived from a HBV preS consensus sequence and are N-terminal preferably acylated and optional C-terminal modified. Di Bisceglie et al., N Engl J Med. 2008; 359(23): 2429-41, relates to a randomized, controlled trial of peginterferon alfa-2a at a dosage of 90 microg per week for 3.5 years, as compared with no treatment, in 1050 patients with chronic hepatitis C and advanced fibrosis who had not had a response to previous therapy with peginterferon and ribavirin. Volz et al., J Hepatol. 2013; 58(5): 861-7, investigates the ability of Myrcludex-B to block HBV spreading post-infection. Heidrich et al., Hepatology. 2014; 60(1): 87-97, relates to a retrospective-prospective follow-up of 77 patients treated for 48 weeks with either PEG-alfa-2a and adefovir (ADV) or either drug alone in the Hep-Net-International-Delta-Hepatitis-Intervention-Study 1 (HIDIT-1) trial. WO 02/094866 relates to an adjuvant and vaccine composition containing pre-S of the hepatitis B virus, and more specifically to a modified recombinant pre-S that can enhance immunogenicity of a general vaccine antigen. Cooksley et al., J Viral Hepat. 2003; 10(4): 298-305, relates to peginterferon alpha-2a (40 kDa) in the treatment of chronic hepatitis B.

### SUMMARY

The present invention is as defined in the appended claims. Any embodiment described below which is not encompassed by these claims is present for illustration purposes only. The claimed invention is based on the finding that a combination of an HBV/HDV pre-S 1 entry inhibitor with an immunomudulator such as interferon results in a decrease or loss of HDV RNA in hepatitis D and/or HBsAg in hepatitis B and D. The claimed invention thus provides a novel combination therapy of HBV/HDV infection, hepatitis B or D, or chronic hepatitis B or D.

The combination or composition may comprise more than one further active ingredient. The composition may further comprise a pharmaceutically acceptable excipient, carrier etc.

The NTCP inhibitor is a pre-S 1 peptide inhibitor, wherein the pre-S 1 peptide inhibitor comprises a pre-S 1 peptide of an HBV virus.

The HBV virus may be any HBV virus, e.g., HBV strain alpha1, HBV strain LSH, woodchuck HBV, Woolly Monkey HBV (WMHBV), HBV subtype *AD, ADR, ADW, ADYW, AR or AYW,* or HBV genotype A to H.

Pre-S1 peptide inhibitors may comprise at least amino acids 2 to 9 and/or 11 to 15 of a pre-S1 peptide of an HBV virus. Alternatively, pre-S1 peptide inhibitors may comprise at least amino acids 9 to 15 or 11 to 15 of a pre-S1 peptide of an HBV virus. Pre-S1 peptide inhibitors may comprise or consist of amino acids 2 to 48 or 2 to 21 of a pre-S1 peptide of an HBV virus.

An exemplary pre-S1 peptide inhibitor comprises the amino acid sequence
between positions 2 and 48 of the HBV pre-S1 consensus sequence:
   GTNL SVPNP LGFFP DHQLD PAFRA NSNNP DWDFN PNKDH WPEAN KVG (SEQ ID NO: 12), or
between positions 2 and 48 of the HBV pre-S1 Genotype C sequence:
   GTNL SVPNP LGFFP DHQLD PAFGA NSNNP DWDFN PNKDH WPEAN QVG (SEQ ID NO: 14), or
between positions 2 and 48 of the HBV pre-S1 Genotype C sequence with an amino acid substitution at position 46 (Gln (Q) --> Lys (K)):
   GTNL SVPNP LGFFP DHQLD PAFGA NSNNP DWDFN PNKDH WPEAN KVG (SEQ ID NO: 13), or
between positions 2 and 48 of the HBV pre-S1 Genotype D sequence:
   GQNL STSNP LGFFP DHQLD PAFRA NTANP DWDFN PNKDT WPDAN KVG (SEQ ID NO: 5).

Pre-S1 peptide inhibitors may be modified by a hydrophobic moiety at the N-terminus. The hydrophobic moiety modification may be by acylation, e.g., acylation with myristoyl or stearoyl.

A preferred pre-S1 peptide inhibitor has the amino acid sequence of GTNL SVPNP LGFFP DHQLD PAFGA NSNNP DWDFN PNKDH WPEAN KVG (SEQ ID NO: 13),
wherein the peptide is modified at the N-terminus by myristoyl.

The preferred pre-S1 peptide inhibitor for use in the invention is Myrcludex B having the following chemical formula:
N-Myristoyl-glycyl-L-threonyl-L-asparaginyl-L-leucyl-L-seryl-L-valyl-L-prolyl-L-asparaginyl-L-prolyl-L-leucyl-glycyl-L-phenylalanyl-L-phenylalanyl-L-prolyl-L-aspartyl-L-histidyl-L-glutaminyl-L-leucyl-L-aspartyl-L-prolyl-L-alanyl-L-phenylalanyl-glycyl-L-alanyl-L-asparaginyl-L-seryl-L-asparaginyl-L-asparaginyl-L-prolyl-L-aspartyl-L-tryptophanyl-L-aspartyl-L-phenylalanyl-L-asparaginyl-L-prolyl-L-asparaginyl-L-lysyl-L-aspartyl-L-histidyl-L-tryptophanyl-L-prolyl-L-glutamyl-L-alanyl-L-asparaginyl-L-lysyl-L-valyl-glycinamide, acetate salt.

Pre-S 1 peptide inhibitors may be modified at the C-terminus or elsewhere to protect the peptide form degradation. Exemplary moieties that can be used for this purpose are D-amino acids, cyclic amino acids, modified amino acids, natural or synthetic polymers such as polyethylene glycol (PEG).

Hereinafter the above NTCP inhibitors, including pre-S 1 peptide inhibitors may be referred to as an inhibitor. This designation includes all NTCP inhibitors, preferably pre-S1 peptide inhibitors, of the invention.

The immunomodulator may be a therapeutic vaccine, an adjuvant or an interferon such as interferon alpha (IFNa), e.g., interferon alpha 2a or interferon alpha 2b. The interferon in the composition for use of the invention is pegylated (PEG-IFN).

If the claimed invention is used with a nucleoside analogue, the nucleoside analogues may be lamivudine, telbivudine or entecavir.

If the claimed invention is used with a nucleotide analogue, the nucleotide analogues may be tenofovir or adefovir.

Hereinafter the above further active ingredients may be referred to as a further active ingredient or a further active agent. This designation includes all nucleoside analogues, nucleotide analogues and immunomodulators, of the disclosure.

Two or more of the above further active ingredients may be used in the combination or composition.

Accordingly, provided are *inter alia* combinations and compositions comprising following ingredients:
a pre-S 1 peptide inhibitor such as Myrcludex B and IFNa or PEG-IFNa;
a pre-S 1 peptide inhibitor such as Myrcludex B and lamivudine;
a pre-S1 peptide inhibitor such as Myrcludex B and telbivudine;
a pre-S1 peptide inhibitor such as Myrcludex B and entecavir;
a pre-S1 peptide inhibitor such as Myrcludex B and tenofovir; or
a pre-S1 peptide inhibitor such as Myrcludex B and adefovir.

A unit dose of Myrcludex B in the combination or composition is be 2 mg, 5 mg, or 10 mg.

A unite dose of interferon or pegylated interferon in the combination or composition is 180 µg.

A unit dose of lamivudine in the combination or composition may be between 10 mg and 100 mg, for example 10 mg, 20 mg, 30 mg, 40 mg, 50 mg, 60 mg, 70 mg, 80 mg, 90 mg or 100 mg, preferably 100 mg.

A unit dose of entecavir in the combination or composition may be between 0.1 mg and 10 mg, for example 0.1 mg, 0.2 mg, 0.3 mg, 0.4 mg, 0.5 mg, 0.6 mg, 0.7 mg, 0.8 mg, 0.9 mg, 1.0 mg, 2.0 mg, 3.0 mg, 4.0 mg, 5.0 mg, 6.0 mg. 7.0 mg, 8.0 mg, 9.0 mg, 10.0 mg, preferably 0.5 mg or 1.0 mg.

A unit dose of telbivudine in the combination or composition may be between 100 mg and 1000 mg, for example 100 mg, 200 mg, 300 mg, 400 mg, 500 mg, 600 mg, 700 mg, 800 mg, 900 mg, or 1000 mg, preferably 500 mg, 600 mg, or 700 mg.

A unite dose of tenofovir in the combination or composition may be between 100 mg and 1000 mg, for example 100 mg, 200 mg, 300 mg, 400 mg, 500 mg, 600 mg, 700 mg, 800 mg, 900 mg, or 1000 mg, preferably 200 mg, 250 mg, 245 mg, or 300 mg.

A unite dose of adefovir in the combination or composition may be between 5 mg to 20 mg, for example 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg, preferably 10 mg.

In another aspect, provided herein is a combination or a composition as defined above for use in a method of treatment of HBV or HDV infection in a subject, hepatitis B, chronic hepatitis B, hepatitis D, or chronic hepatitis D.

In another aspect, provided herein is a pre-S1 peptide inhibitor for use in a method of treatment of HBV or HDV infection, hepatitis B, chronic hepatitis B, hepatitis D, or chronic hepatitis D in a subject, wherein the method comprises the administration of a further active ingredient, wherein the further active ingredient is selected from a group consisting of an immunomodulator, preferably interferon; a nucleotide analogue, preferably tenofovir or adefovir; and an nucleoside analogue, preferably lamivudine, telbivudine and entecavir.

In another aspect, provided herein is a method of treatment of HBV or HDV infection, hepatitis B, chronic hepatitis B, hepatitis D, or chronic hepatitis D in a subject, comprising administering to the subject an inhibitor of Na+-taurocholate cotransporting polypeptide (NTCP), preferably a pre-S1 peptide inhibitor, and a further active ingredient selected from the group consisting of a nucleotide analogue, a nucleoside analogue and an immunomodulator.

Said inhibitor and said further active ingredient may be provided as a combination or a composition as defined above. More than one further active ingredient may be used in the methods.

Thus, the methods may, for example, comprise administering:
a pre-S1 peptide inhibitor such as Myrcludex B and IFNa or PEG-IFNa;
a pre-S1 peptide inhibitor such as Myrcludex B and lamivudine;
a pre-S1 peptide inhibitor such as Myrcludex B and telbivudine;
a pre-S1 peptide inhibitor such as Myrcludex B and entecavir;
a pre-S1 peptide inhibitor such as Myrcludex B and tenofovir; or
a pre-S1 peptide inhibitor such as Myrcludex B and adefovir.

The method may comprise:
administering a pre-S1 peptide inhibitor in a dose of 2 mg, 5 mg or 10 mg per day for HDV and HBV infection,
   and:
   administering pegylated interferon in the dose of 180 µg per week. A weekly dose may be administered once a week or several times a week, for example twice or three times per week or every day with doses determined such as to sum up in said weekly dose.
and optionally further administering one or more of the following further active ingredients lamivudine in a dose of between 10 mg and 100 mg per day, for example 10 mg, 20 mg, 30 mg, 40 mg, 50 mg, 60 mg, 70 mg, 80 mg, 90 mg or 100 mg per day, preferably 100 mg per day;
entecavir in a dose of between 0.1 mg and 10 mg per day, for example 0.1 mg, 0.2 mg, 0.3 mg, 0.4 mg, 0.5 mg, 0.6 mg, 0.7 mg, 0.8 mg, 0.9 mg, 1.0 mg,2.0 mg, 3.0 mg, 4.0 mg, 5.0 mg, 6.0 mg. 7.0 mg, 8.0 mg, 9.0 mg, 10.0 mg per day, preferably 0.5 mg or 1.0 mg per day;
telbivudine in a dose of between 100 mg and 1000 mg per day, for example 100 mg, 200 mg, 300 mg, 400 mg, 500 mg, 600 mg, 700 mg, 800 mg, 900 mg, or 1000 mg per day, preferably 500 mg, 600 mg, or 700 mg per day;
administering tenofovir in a dose of between 100 mg and 1000 mg per day, for example 100 mg, 200 mg, 300 mg, 400 mg, 500 mg, 600 mg, 700 mg, 800 mg, 900 mg, or 1000 mg per day, preferably 200 mg, 250 mg, 245 mg, or 300 mg per day;
administering adefovir is administered in a dose of between 5 mg to 20 mg per day, for example 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg per day, preferably 10 mg per day.

An inhibitor and a further active ingredient may be administered sequentially. For example, an inhibitor may be administered for at least one cycle or a course comprising one or more cycles (such as 2, 3, 4, 5, 6, 7, 8, 9 or 10 cycles) followed by the administration of a further active ingredient for at least one cycle or a course comprising one or more cycles (such as 2, 3, 4, 5, 6, 7, 8, 9 or 10 cycles). The duration of one cycle may be 1, 2, 3, 4, 5, 6, 7, 8, 9, 10, 11, or 12 weeks. The duration of one cycle may be at least 1, 2, 3, 4, 5, 6, 7, 8, 9, 10, 11, or 12 weeks. The duration of one course may be 12 weeks, 24 weeks, 36 weeks, 48 weeks, 60 weeks, 1 year, 1.1 years, 1.2 years, 1.3 years, 1.4 years, 1.5 years, 1.6 years, 1.7 years, 1.8 years, 1.8 years, 1.9 years, or 2.0 years, or 3 years, or 4 years.

An inhibitor and a further active ingredient may be administered concurrently/concomitantly. According to this administration schedule, the administration of an inhibitor timely overlaps with the administration of a further active ingredient. The duration of the administration of an inhibitor and a further active ingredient may be identical or essentially identical. For example, both an inhibitor and a further active ingredient may be administered for at least one cycle or a course comprising one or more cycles (such as 2, 3, 4, 5, 6, 7, 8, 9 or 10 cycles). The duration of one cycle may be 1, 2, 3, 4, 5, 6, 7, 8, 9, 10, 11, or 12 weeks. The duration of one cycle may be at least 1, 2, 3, 4, 5, 6, 7, 8, 9, 10, 11, or 12 weeks. The duration of one course may be 12 weeks, 24 weeks, 36 weeks, 48 weeks, 60 weeks, 1 year, 1.1 years, 1.2 years, 1.3 years, 1.4 years, 1.5 years, 1.6 years, 1.7 years, 1.8 years, 1.8 years, 1.9 years, or 2.0 years, or 3 years, or 4 years. For example, a 24 weeks course of Myrcludes B may be administered at the same time as a 24 weeks course of PEG-IFNa. In this administration scheme, Myrcludex B may be administered daily, whereas PEG-IFNa may be administered weekly. In this administration schedule, an inhibitor and a further active ingredient may be administered simultaneously, for example at essentially the same time or in a single composition.

An inhibitor and a further active ingredient maybe delivered by various delivery routes, depending on the type of ingredient. Administration routs include enteral route (e.g., orally and rectally), parenteral route (e.g. intravenously, intramuscularly, subcutaneously intraperitoneally), topically. Preferably, the pre-S1 peptide inhibitor is delivered subcutaneously. Preferably, PEG-IFNa is delivered subcutaneously.

The method may be used for human treatment.

### DESCRIPTION OF THE FIGURES

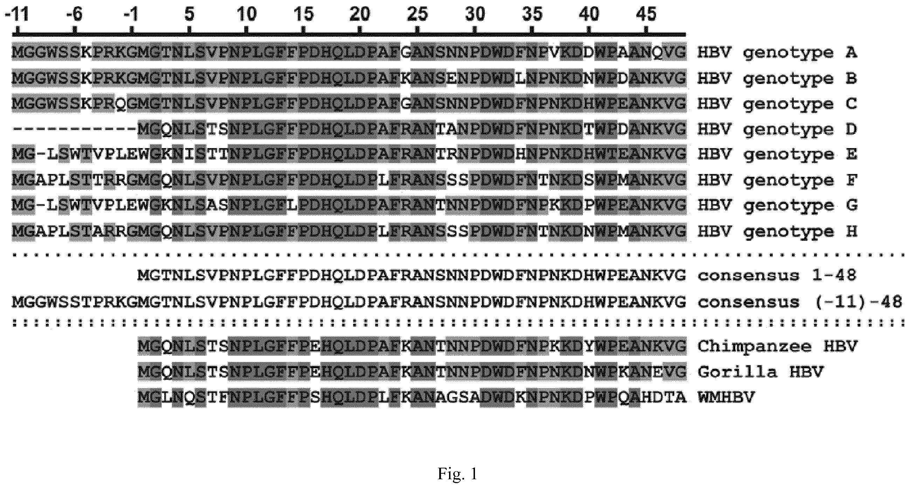

**Figure** 1. Sequence alignment of the human HBV genotypes A to H and the chimpanzee, gorilla, and woolly monkey hepatitis B viruses. The short (residues 2 to 48) and long (residues -11 to 48) consensus sequences derived from HBV genotypes A to H are depicted in the middle. Identical amino acids are marked by dark-gray boxes. Non-identical amino acids used for the consensus sequences are marked by light-gray boxes.

### DETAILED DESCRIPTION OF THE INVENTION

The present invention is as defined in the appended claims. Any embodiment described below which is not encompassed by these claims is present for illustration purposes only. The claimed invention is based on the finding that a combination of an HBV/HDV pre-S1 entry inhibitor with an immunomudulator such as interferon results in a decrease or loss of HDV RNA in hepatitis D and/or HBsAg in hepatitis B and D.

In one aspect, the claimed invention relates to combinations and compositions, preferably pharmaceutical compositions, comprising an inhibitor of Na+-taurocholate cotransporting polypeptide (NTCP) and an immunomodulator. An NTCP inhibitor may inhibit the HBV/HDV cell entry and may also be referred to as an entry inhibitor.

The combination or compositions may comprise more than one further active ingredient. The compositions may further comprise a pharmaceutically acceptable excipient, carrier etc. The composition may also be referred to as a pharmaceutical composition.

In a combination, the individual active ingredients, i.e., said inhibitor of Na+-taurocholate cotransporting polypeptide (NTCP) and said active ingredient or ingredients are provided in separate vials. In a composition, the individual active ingredients, i.e., said inhibitor of Na+taurocholate cotransporting polypeptide (NTCP) and said active ingredient are provided in a single vial.

HBV/HDV entry inhibitor is a compound capable of inhibiting viral entry into a cell such as a hepatocyte. In particular, an HBV/HDV entry inhibitor is capable of binding and inhibiting NTCP receptor on cell surface. Thus, an HBV/HDV entry inhibitor is preferably an NTCP inhibitor.

In one embodiment, an HBV/HDV entry inhibitor may be derived from N-terminal domain of the large (L) HBV surface protein, pre-S1, of any HBV virus, in particular of any HBV strain, genotype or subtype. Examples of HBV strains are HBV strain alpha1, HBV strain LSH (chimpanzee isolate), woodchuck HBV, Woolly Monkey HBV (WMHBV). Examples of HBV subtypes *AD, ADR, ADW, ADYW, AR* and *AYW.* Examples of HBV genotypes are genotypes A to H of human HBV. According to the standard HBV nomenclature, pre-S1 peptide has amino acid coordinates between -11 and 108.

An amino acid sequence alignment of pre-S1 peptides between positions -11 and 48 of various human genotypes as well as respective sequences from pre-S1 peptides of hepatitis B viruses isolated from chimpanzee, gorilla, and woolly monkey is provided in Figure 1. The alignment of the HBV genotypes A to H shows that compared to genotype D, all other genotypes contain an additional 10 (E and G) or 11 (A, B, C, F, and H) N-terminal pre-S1 amino acids. Accordingly, a viral pre-S1 peptide may have different amino acid coordinates and hence a different length, depending on the viral stain, genotype or subtype.

The sequence alignment also indicates that pre-S1 peptides of various genotypes share conserved regions along the peptide length. A highly conserved region is situated between amino acid positions 9 to 21. In addition, individual highly conserved amino acids are situated between amino acids 2 and 6. These conserved sequences are also present in the hepatitis B viruses isolated from chimpanzee, gorilla, and woolly monkey.

Experimental evidence indicate that the capacity of synthetic HBV pre-S I-derived peptides to interfere with the HBV infection depends on the presence of some amino acids within conserved the preS-1 regions (Schulze et al., 2010). Thus, of importance for the peptide activity is a region between amino acid positions 11 and 15 of pre-S1 and in particular amino acids 11, 12, 13, 14 and/or 15 within this region. Further, replacement of some amino acids within a region between positions 2 and 9 are reduced the HBV inhibitory activity of a pre-S1 peptide, whereby amino acid 9 appears essential. Further regions that may contribute to the inhibitory activity of HBV pre-S1-derived peptides are amino acids 2 to 8, 16 to 20, and, to a less pronounced extent, 34 to 48.

Thus, pre-S1 peptide-derived HBV/HDV entry inhibitors (hereinafter referred to as pre-S1 peptide inhibitors) may span amino acids -11 to 108 of HBV pre-S1 (entire HBV pre-S1) or any portion within this region. Hereinafter reference will be made to the standard HBV amino acid numbering. In particular, pre-S1 peptide inhibitor may span amino acids -11 to 78. In some genotypes, the first 10 (genotypes E and G) or 11 (genotypes A, B, C, F, and H) amino acids are absent. Accordingly, pre-S1 peptide inhibitor may span amino acids 1 to 78 of HBV. One or more amino acids beyond amino acid 48, i.e., from amino acid 49 to 108 may be absent. Accordingly, pre-S1 peptide inhibitor may span amino acids -11 to 48. Within the range of -11 to 48, the first 1, 2, 3, 4, 5, 6, 7, 8, 9, 10 or 11 amino acids may be dispensed with. Accordingly, pre-S1 peptide inhibitor may span amino acids 1 to 48. Amino acid 1 (Met) may be absent. Respective pre-S1 peptide inhibitor may span amino acids 2 to 48 and optionally comprise further amino acids at the C-terminus. Within the region of 2 to 48, amino acids 2 to 21 and 34 to 48 are more important for pre-S1 peptide inhibitor function than amino acids 21 to 33. Amino acids 9 to 15, in particular 11 to 15 are most important. Accordingly, pre-S1 peptide inhibitor may consist of amino acids 2 to 48 or a truncated portion thereof. Alternatively, pre-S1 peptide inhibitor may comprise amino acids 2 to 48 or a truncated portion thereof.

A truncated portion may be obtained by amino acid deletions from either end of pre-S1 peptide and have a length of between 46 and 5 amino acids. In particular, a truncated portion may have a length of 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6 or 5 amino acids.

A truncated portion preferably comprises amino acids 9 to 15, 10 to 15, or 11 to 15. A truncated portion preferably consists of amino acids 11 to 15. A truncated portion may gradually be extended by addition of amino acids flanking acids 11 to 15. For example, a truncated portion may consists of amino acids 2 to 15, 3 to 15, 4 to 15, 5 to 15, 6 to 15, 7 to 15, 8 to 15, 9 to 15 or 10 to 15. Any of amino acids 16 to 48 may be used in this example to provide further truncated portions.

In another example, a truncated portion may consist of amino acids 2 to 15, 2 to 16, 2 to 17, 2 to 18, 2 to 19, 2 to 20, 2 to 21, 2 to 22, 2 to 23, 2 to 24, 2 to 25, 2 to 26, 2 to 27, 2 to 28, 2 to 29, 2 to 30, 2 to 31, 2 to 32, 2 to 33, 2 to 34, 2 to 35, 2 to 36, 2 to 37, 2 to 38, 2 to 39, 2 to 40, 2 to 41, 2 to 42, 2 to 43, 2 to 44, 2 to 45, 2 to 45, 2 to 46, 2 to 47 or 2 to 48. Any of amino acids 3 to 11 may be used in this example to provide further truncated portions.

Further examples of truncated portions are amino acids 9 to 15, 2 to 21, 5 to 21, 2 to 15, 2 to 20, 2 to 25, 2 to 30, 2 to 35, 2 to 40.

A truncated portion of pre-S1 peptide may be a functional fragment of pre-S1 peptide retaining the function of pre-S1 peptide in inhibiting HBV cell entry, binding and/or inhibiting NTCT, an essential HBV receptor.

Pre-S1 peptide inhibitors are derived from pre-S1 peptides of various HBV viruses, e.g., various genotypes, strains or subtypes. In particular, pre-S1 peptide inhibitors may be derived from pre-S 1 of genotypes A, B, C, D, E, F, G or H, or of subtypes *AD, ADR, ADW, ADYW, AR* and *AYW.* Pre-S1 peptides from various HBV viruses will usually be homologues. Homologues from various species have structural and functional similarity and usually share a common evolutionary ancestor. It is envisioned that pre-S1 homologues from further genotypes, strains or subtypes to be identified in the future will be equally suitable as pre-S1 peptide inhibitors.

Pre-S1 peptide inhibitors may be derived from a pre-S1 consensus sequence (Figure 1).

Examples of HBV pre-S1 sequences between amino acids -11 or -10 or 1 (depending on the genotype) and 48 of various genotypes and the consequence sequence are provided below:
HBV preS1 consensus sequence (positions (-11) to 48)
   (-11)-M GGWSS TPRKG MGTNL SVPNP LGFFP DHQLD PAFRA NSNNP DWDFN PNKDH WPEAN KVG-48 (SEQ ID NO: 1)
HBV preS1 Genotype A sequence (positions (-11) to 48)
   (-11)-M GGWSS KPRKG MGTNL SVPNP LGFFP DHQLD PAFGA NSNNP DWDFN PVKDD WPAAN QVG-48 (SEQ ID NO: 2)
HBV preS1 Genotype B sequence (positions (-11) to 48)
   (-11)-M GGWSS KPRKG MGTNL SVPNP LGFFP DHQLD PAFKA NSENP DWDLN PHKDN WPDAN KVG-48 (SEQ ID NO: 3)
HBV preS1 Genotype C sequence (positions (-11) to 48)
   (-11)-M GGWSS KPRQG MGTNL SVPNP LGFFP DHQLD PAFGA NSNNP DWDFN PNKDH WPEAN QVG-48 (SEQ ID NO: 4)
HBV preS1 Genotype D sequence (positions 1 to 48)
   1-MGQNL STSNP LGFFP DHQLD PAFRA NTANP DWDFN PNKDT WPDAN KVG-48 (SEQ ID NO: 5)
HBV preS1 Genotype E sequence (positions (-10) to 48)
   (-10)-MGLSW TVPLE WGKNI STTNP LGFFP DHQLD PAFRA NTRNP DWDHN PNKDH WTEAN KVG-48 SEQ ID NO: 6)
HBV preS1 Genotype F sequence (positions (-11) to 48)
   (-11)-M GAPLS TTRRG MGQNL SVPNP LGFFP DHQLD PLFRA NSSSP DWDFN TNKDS WPMAN KVG-48 (SEQ ID NO: 7)
HBV preS1 Genotype G sequence (positions (-10) to 48)
   (-10)-MGLSW TVPLE WGKNL SASNP LGFLP DHQLD PAFRA NTNNP DWDFN PKKDP WPEAN KVG-48 (SEQ ID NO: 8)
HBV preS1 Genotype H sequence (positions (-11) to 48)
   (-11)-M GAPLS TARRG MGQNL SVPNP LGFFP DHQLD PLFRA NSSSP DWDFN TNKDN WPMAN KVG-48 (SEQ ID NO: 9)

The pre-S1 sequence between amino acids 1 and 48 from Woolly Monkey WMHBV is provided below:
1-MGLNQ STFNP LGFFP SHQLD PLFKA NAGSA DWDKN PNKDP WPQAH DTA (SEQ ID NO: 10)

Pre-S1 peptide inhibitors may contain one or more (e.g., 1, 2, 3, 4, 5, 6, 7,8 9, or 10) amino acid substitutions which do not substantially reduce their HBV inhibitory activity. Preferably, HBV inhibitory activity should not be reduced by more than two orders of magnitude. In particular, reduction by 2-, 3-, 4-, 5-, 6-, 7-, 8-, 9-, 10-, 11-, 12-, 13-, 14-, 15-, 16-, 17-, 18-, 19-, or 20-fold is tolerated. The HBV inhibitory activity may be measured in terms of IC₅₀ or IC₉₀. Amino acid substitutions may be conservative or non-conservative. Amino acid substitutions preferably do not affect amino acids important for the HBV inhibitory activity of pre-S1 peptide inhibitors. Individual amino acids located within a highly conserved part of pre-S1 spanning amino acids 2 to 21 are more important for the activity than amino acids outside this region. Within this region, amino acids 9 to 15 are even more important. Among these, amino acids 9, 11, 12 and 13 are most important. Accordingly, amino acid substitutions are preferably located outside amino acids 11 to 15, 9 to 15, 5 to 15, or 2 to 15. Amino acid substitutions may also be located outside amino acids 2 to 8, 16 to 20 or 34 to 48. Amino acid substitutions may be located within amino acids 20 to 23, 26 to 32.

An example of a pre-S1 sequence derived from the pre-S1 sequence of genotype C and containing an amino acid substitution at position 46 (Gln (Q) --> Lys (K)) is provided below:
(-11)-M GGWSS KPRQG MGTNL SVPNP LGFFP DHQLD PAFGA NSNNP DWDFN PNKDH WPEAN KVG-48 (SEQ ID NO: 11).

A preferred peptide inhibitor is based on pre-S1 peptide between positions 2 and 48 (according to the standard HBV nomenclature) of the amino acid sequence of the HBV pre-S1 consensus sequence:
GTNL SVPNP LGFFP DHQLD PAFRA NSNNP DWDFN PNKDH WPEAN KVG (SEQ ID NO: 12).

Another preferred peptide inhibitor is based on pre-S1 peptide between positions 2 and 48 (according to the standard HBV nomenclature) of the amino acid sequence of the HBV preS1 Genotype C:
GTNL SVPNP LGFFP DHQLD PAFGA NSNNP DWDFN PNKDH WPEAN QVG (SEQ ID NO: 14)

A most preferred peptide inhibitor is based on pre-S1 peptide between positions 2 and 48 (according to the standard HBV nomenclature) of the amino acid sequence of genotype C with an amino acid substitution at position 46 (Gln (Q) --> Lys (K))
GTNL SVPNP LGFFP DHQLD PAFGA NSNNP DWDFN PNKDH WPEAN KVG (SEQ ID NO: 13).

A pre-S1 peptide inhibitor may be an N-terminally or C-terminally truncated portion of the above pre-S1 inhibitors of at least 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, or 46 amino acids. The portion retains its functionality as NTCP inhibitor, NTCP binding protein or HBV/HDV entry inhibitor.

Pre-S1 peptide inhibitors may have genotype-spanning inhibitory activity, i.e. they may crossinhibit genotypes other than the authentic genotype from which the pre-S1 peptide is derived.

Pre-S1 peptide inhibitors are preferably hydrophobic. They may be modified a hydrophobic moiety. Hydrophobic modification by acylation is preferred. Acylation may be acylation with carboxylic acids, fatty acids, or amino acids with lipophilic side chains. Alternatively, pre-S1 peptide inhibitors may be modified by cholesterol, derivatives of cholesterol, phospholipids, glycolipids, glycerol esters, steroids, ceramids, isoprene derivatives, adamantine, farnesol, aliphatic groups, or polyaromatic compounds. Fatty acids may be saturated or unsaturated fatty acids, branched or unbranched fatty acids, preferably with 8 to 22 carbon atoms. Examples of suitable fatty acids for acylation are myristic acid (D14), stearic acid (C18), palmitic acid (C16). Variation of hydrophobic moiety allows for the modulation of specific pre-S1 peptide activities.

A hydrophobic moiety is preferably attached to the N-terminus of the pre-S1 peptide inhibitor. Thus, a hydrophobic moiety may be attached to the N-terminal amino acid of the pre-S 1 peptide inhibitor, or to an amino acid in close proximity to the N-terminus, e.g. amino acids -5, -4, -3, -2, -1, 1, 2, 3, 4, or 5. More than one hydrophobic moiety may be used for the modification of pre-S1 peptide inhibitor. The hydrophobic moieties can be identical or different. The attachment of the hydrophobic moieties is preferably by covalent binding, which can be achieved via carbamate, amide, ether, disulfide or any other linkage that is within the skill of the person skilled in the art.

A preferred peptide inhibitor is a pre-S1 peptide of amino acid sequence GTNL SVPNP LGFFP DHQLD PAFGA NSNNP DWDFN PNKDH WPEAN KVG (SEQ ID NO: 13),
which is modified at the N-terminus by myristoyl or stearoyl.

The chemical name of the pre-S1 peptide inhibitor Myrcludex B is
N-Myristoyl-glycyl-L-threonyl-L-asparaginyl-L-leucyl-L-seryl-L-valyl-L-prolyl-L-asparaginyl-L-prolyl-L-leucyl-glycyl-L-phenylalanyl-L-phenylalanyl-L-prolyl-L-aspartyl-L-histidyl-L-glutaminyl-L-leucyl-L-aspartyl-L-prolyl-L-alanyl-L-phenylalanyl-glycyl-L-alanyl-L-asparaginyl-L-seryl-L-asparaginyl-L-asparaginyl-L-prolyl-L-aspartyl-L-tryptophanyl-L-aspartyl-L-phenylalanyl-L-asparaginyl-L-prolyl-L-asparaginyl-L-lysyl-L-aspartyl-L-histidyl-L-tryptophanyl-L-prolyl-L-glutamyl-L-alanyl-L-asparaginyl-L-lysyl-L-valyl-glycinamide, acetate salt.

An abbreviated chemical name of Myrcludex B is Myristoyl-Gly-Thr-Asn-Leu-Ser-Val-Pro-Asn-Pro-Leu-Gly-Phe-Phe-Pro-Asp-His-Gln-Leu-Asp-Pro-Ala-Phe-Gly-Ala-Asn-Ser-Asn-Asn-Pro-Asp-Trp-Asp-Phe-Asn-Pro-Asn-Lys-Asp-His-Trp-Pro-Glu-Ala-Asn-Lys-Val-Gly-NH2, acetate salt.

Pre-S1 peptide inhibitors may further be modified in a conventional way to improve peptide stability, e.g. stability against degradation. Such modification may include a modification with amide, D-amino acid, modified amino acid, cyclic amino acid, natural polymer, synthetic polymer, or glycine.

The further active ingredient is an immunomodulator.

In a further embodiment, an HBV/HDV entry inhibitor may be a small molecule.

Nucleoside analogues are nucleosides which contain a nucleic acid analogue and a sugar. Examples of nucleoside analogues used in the treatment of HBV or HDV are lamivudine, telbivudine and entecavir. Other nucleoside analogues may be used in the practice of the invention.

Nucleotide analogues are nucleotides which contain a nucleic acid analogue, a sugar and one to three phosphate groups. Examples of nucleotide analogues used in the treatment of HBV or HDV are tenofovir and adefovir. Other nucleotide analogues may be used in the practice of the invention.

Example of immunomodulators, i.e. active ingredients that have the ability to modulate the activity of the immune system of a subject, are interferon, therapeutic vaccine, and adjuvant.

Interferon may be interferon alpha, e.g., interferon alpha 2a or interferon alpha 2b. The activity of interferon alpha 2a and interferon alpha 2b may be similar in HBV and HDV infection.

The interferon is pegylated, i.e. attached to a polyethylene glycol (PEG) moiety. Pegylation may improve pharmacokinetics and convenience for the patient, but does not influence mechanism of action of interferon. In addition, the activity of interferon alpha 2a and 2b is very similar in HBV and HDV infection.

Accordingly, provided are *inter alia* combinations and compositions comprising following ingredients:
a pre-S1 peptide inhibitor such as Myrcludex B and IFNa or PEG-IFNa;
a pre-S1 peptide inhibitor such as Myrcludex B and lamivudine;
a pre-S1 peptide inhibitor such as Myrcludex B and telbivudine;
a pre-S1 peptide inhibitor such as Myrcludex B and entecavir;
a pre-S1 peptide inhibitor such as Myrcludex B and tenofovir; or
a pre-S1 peptide inhibitor such as Myrcludex B and adefovir.

A unit dose of a pre-S1 peptide inhibitor such as Myrcludex B in the combination or composition is 2 mg, 5 mg, or 10 mg.

A unite dose of interferon or pegylated interferon in the combination or composition is 180 µg.

A unit dose of lamivudine in the combination or composition may be between 10 mg and 100 mg, for example 10 mg, 20 mg, 30 mg, 40 mg, 50 mg, 60 mg, 70 mg, 80 mg, 90 mg or 100 mg, preferably 50 mg or 100 mg.

A unit dose of entecavir in the combination or composition may be between 0.1 mg and 10 mg, for example 0.1 mg, 0.2 mg, 0.3 mg, 0.4 mg, 0.5 mg, 0.6 mg, 0.7 mg, 0.8 mg, 0.9 mg, 1.0 mg, 2.0 mg, 3.0 mg, 4.0 mg, 5.0 mg, 6.0 mg. 7.0 mg, 8.0 mg, 9.0 mg, or 10.0 mg, preferably 0.5 mg or 1.0 mg.

A unit dose of telbivudine in the combination or composition may be between 100 mg and 1000 mg, for example 100 mg, 200 mg, 300 mg, 400 mg, 500 mg, 600 mg, 700 mg, 800 mg, 900 mg, or 1000 mg, preferably 500 mg, 600 mg, or 700 mg.

A unite dose of tenofovir in the combination or composition may be between 100 mg and 1000 mg, for example 100 mg, 200 mg, 300 mg, 400 mg, 500 mg, 600 mg, 700 mg, 800 mg, 900 mg, or 1000 mg, preferably 200 mg, 250 mg, 245 mg, or 300 mg.

A unite dose of adefovir in the combination or composition may be between 5 mg to 20 mg, for example 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg, preferably 10 mg.

An exemplary composition or combination comprises:
1. 2 mg pre-S1 peptide inhibitor such as Myrcludex B and 180 µg interferon such as pegylated interferon alpha (PEG-IFNa). The amount of interferon may also vary between 10 µg and 300 µg and be for example 10 µg, 30 µg, 50 µg, 70 µg, 90 µg, 100 µg, 120 µg, 130 µg, 140 µg, 150 µg, 160 µg, 170 µg, 180 µg, 190 µg, 200 µg, 210 µg, 220 µg, 230 µg, 240 µg, 250 µg, 260 µg, 270 µg, 280 µg, 290 µg, or 300 µg.
2. 5 mg pre-S1 peptide inhibitor such as Myrcludex B and 180 µg interferon such as pegylated interferon alpha (PEG-IFNa). The amount of interferon may also vary between 10 µg and 300 µg and be for example 10 µg, 30 µg, 50 µg, 70 µg, 90 µg, 100 µg, 120 µg, 130 µg, 140 µg, 150 µg, 160 µg, 170 µg, 180 µg, 190 µg, 200 µg, 210 µg, 220 µg, 230 µg, 240 µg, 250 µg, 260 µg, 270 µg, 280 µg, 290 µg, or 300 µg.
3. 10 mg pre-S1 peptide inhibitor such as Myrcludex B and 180 µg interferon such as pegylated interferon alpha (PEG-IFNa). The amount of interferon may also vary between 10 µg and 300 µg and be for example 10 µg, 30 µg, 50 µg, 70 µg, 90 µg, 100 µg, 120 µg, 130 µg, 140 µg, 150 µg, 160 µg, 170 µg, 180 µg, 190 µg, 200 µg, 210 µg, 220 µg, 230 µg, 240 µg, 250 µg, 260 µg, 270 µg, 280 µg, 290 µg, or 300 µg.
4. 2 mg pre-S1 peptide inhibitor such as Myrcludex B and 100 mg lamivudine. The amount of lamivudine may also vary between 10 and 100 mg, and be for example 10 mg, 20 mg, 30 mg, 40 mg, 50 mg, 60 mg, 70 mg, 80 mg, 90 mg or 100 mg
5. 5 mg pre-S1 peptide inhibitor such as Myrcludex B and 100 mg lamivudine. The amount of lamivudine may also vary between 10 and 100 mg, and be for example 10 mg, 20 mg, 30 mg, 40 mg, 50 mg, 60 mg, 70 mg, 80 mg, 90 mg or 100 mg.
6. 10 mg pre-S1 peptide inhibitor such as Myrcludex B and 100 mg lamivudine. The amount of lamivudine may also vary between 10 and 100 mg, and be for example 10 mg, 20 mg, 30 mg, 40 mg, 50 mg, 60 mg, 70 mg, 80 mg, 90 mg or 100 mg.
7. 2 mg pre-S1 peptide inhibitor such as Myrcludex B and 0.5 mg or 1.0 mg entecavir. The amount of entecavir may also vary between 0.1 and 10 mg and be for example 0.1 mg, 0.2 mg, 0.3 mg, 0.4 mg, 0.5 mg, 0.6 mg, 0.7 mg, 0.8 mg, 0.9 mg, 1.0 mg, 2.0 mg, 3.0 mg, 4.0 mg, 5.0 mg, 6.0 mg. 7.0 mg, 8.0 mg, 9.0 mg, or 10.0 mg.
8. 5 mg pre-S1 peptide inhibitor such as Myrcludex B and 0.5 mg or 1.0 mg entecavir. The amount of entecavir may also vary between 0.1 and 10 mg and be for example 0.1 mg, 0.2 mg, 0.3 mg, 0.4 mg, 0.5 mg, 0.6 mg, 0.7 mg, 0.8 mg, 0.9 mg, 1.0 mg, 2.0 mg, 3.0 mg, 4.0 mg, 5.0 mg, 6.0 mg. 7.0 mg, 8.0 mg, 9.0 mg, or 10.0 mg.
9. 10 mg pre-S1 peptide inhibitor such as Myrcludex B and 0.5 mg or 1.0 mg entecavir. The amount of entecavir may also vary between 0.1 and 10 mg and be for example 0.1 mg, 0.2 mg, 0.3 mg, 0.4 mg, 0.5 mg, 0.6 mg, 0.7 mg, 0.8 mg, 0.9 mg, 1.0 mg, 2.0 mg, 3.0 mg, 4.0 mg, 5.0 mg, 6.0 mg. 7.0 mg, 8.0 mg, 9.0 mg, or 10.0 mg.
10. 2 mg pre-S1 peptide inhibitor such as Myrcludex B and 600 mg telbivudine. The amount of telbivudine may also vary between 100 and 1000 mg, and be for example 100 mg, 200 mg, 300 mg, 400 mg, 500 mg, 600 mg, 700 mg, 800 mg, 900 mg, or 1000 mg.
11. 5 mg pre-S1 peptide inhibitor such as Myrcludex B and 600 mg telbivudine. The amount of telbivudine may also vary between 100 and 1000 mg, and be for example 100 mg, 200 mg, 300 mg, 400 mg, 500 mg, 600 mg, 700 mg, 800 mg, 900 mg, or 1000 mg.
12. 10 mg pre-S1 peptide inhibitor such as Myrcludex B and 600 mg telbivudine. The amount of telbivudine may also vary between 100 and 1000 mg, and be for example 100 mg, 200 mg, 300 mg, 400 mg, 500 mg, 600 mg, 700 mg, 800 mg, 900 mg, or 1000 mg.
13. 2 mg pre-S1 peptide inhibitor such as Myrcludex B and 200 mg, 245 mg, or 300 mg tenofovir. The amount of tenofovir may also vary between 100 and 1000 mg, and be for example 100 mg, 200 mg, 300 mg, 400 mg, 500 mg, 600 mg, 700 mg, 800 mg, 900 mg, or 1000 mg.
14. 5 mg pre-S1 peptide inhibitor such as Myrcludex B and 200 mg, 245 mg, or 300 mg tenofovir. The amount of tenofovir may also vary between 100 and 1000 mg, and be for example 100 mg, 200 mg, 300 mg, 400 mg, 500 mg, 600 mg, 700 mg, 800 mg, 900 mg, or 1000 mg.
15. 10 mg pre-S1 peptide inhibitor such as Myrcludex B and 200 mg, 245 mg, or 300 mg tenofovir. The amount of tenofovir may also vary between 100 and 1000 mg, and be for example 100 mg, 200 mg, 300 mg, 400 mg, 500 mg, 600 mg, 700 mg, 800 mg, 900 mg, or 1000 mg.
16. 2 mg pre-S1 peptide inhibitor such as Myrcludex B and 100 mg adefovir. The amount of adefovir may also vary between 5 to 20 mg, and be for example 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg.
17. 5 mg pre-S1 peptide inhibitor such as Myrcludex B and 100 mg adefovir. The amount of adefovir may also vary between 5 to 20 mg, and be for example 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg.
18. 10 mg pre-S1 peptide inhibitor such as Myrcludex B and 100 mg adefovir. The amount of adefovir may also vary between 5 to 20 mg, and be for example 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg.

The above combinations and compositions may be used in the methods of treatment of HBV or HDV infection, hepatitis B or hepatitis D.

In another aspect, the claimed invention relates to the treatment of HBV/HDV infections, hepatitis B and D, and chronic hepatitis B and D by a combination of an NTCP inhibitor / HBV/HDV entry inhibitor and an immunomodulator. This combination therapy leads to a viral RNA decline any eventually results in a decline of HBsAg levels or loss of HBsAg which is a sign of complete cure and hence the ultimate goal of HBV/HDV therapy.

Said inhibitor and said further active ingredient are provided as a combination or a composition as defined above. More than one further active ingredient may be used in the method.

Thus, the method may, for example, comprise administering to an individual in need of HBV/HDV therapy:
a pre-S1 peptide inhibitor such as Myrcludex B and IFNa or PEG-IFNa;
a pre-S1 peptide inhibitor such as Myrcludex B and lamivudine;
a pre-S1 peptide inhibitor such as Myrcludex B and telbivudine;
a pre-S1 peptide inhibitor such as Myrcludex B and entecavir;
a pre-S1 peptide inhibitor such as Myrcludex B and tenofovir; or
a pre-S1 peptide inhibitor such as Myrcludex B and adefovir.

The method may comprise:
administering a pre-S1 peptide inhibitor in a dose of 2 mg, 5 mg or 10 mg per day for HDV and HBV infection,
and:
interferon such as pegylated interferon alpha (PEG-IGNa) at the dose of 180 µg per week. A weekly dose may be administered once a week or several times a week, for example twice or three times per week or every day with doses determined such as to sum up in said weekly dose.
and optionally further administering one or more of the following further active ingredients:
   lamivudine at a dose of between 10 mg and 100 mg per day, for example 10 mg, 20 mg, 30 mg, 40 mg, 50 mg, 60 mg, 70 mg, 80 mg, 90 mg or 100 mg per day, preferably 100 mg per day;
   entecavir at a dose of between 0.1 mg and 10 mg per day, for example 0.1 mg, 0.2 mg, 0.3 mg, 0.4 mg, 0.5 mg, 0.6 mg, 0.7 mg, 0.8 mg, 0.9 mg, 1.0 mg, 2.0 mg, 3.0 mg, 4.0 mg, 5.0 mg, 6.0 mg. 7.0 mg, 8.0 mg, 9.0 mg, 10.0 mg per day, preferably 0.5 mg or 1.0 mg per day;
   telbivudine at a dose of between 100 and 1000 mg per day, for example 100 mg, 200 mg, 300 mg, 400 mg, 500 mg, 600 mg, 700 mg, 800 mg, 900 mg, or 1000 mg per day, preferably 500 mg, 600 mg, or 700 mg per day;
   tenofovir at a dose of between 100 mg and 1000 mg per day, for example 100 mg, 200 mg, 300 mg, 400 mg, 500 mg, 600 mg, 700 mg, 800 mg, 900 mg, or 1000 mg per day, preferably 200 mg, 250 mg, 245 mg, or 300 mg per day;
   adefovir at a dose of between 5 mg to 20 mg per day, for example 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg per day, preferably 10 mg per day; and/or

The method may comprise administering:
1. 2 mg pre-S1 peptide inhibitor such as Myrcludex B and 180 µg interferon such as pegylated interferon alpha (PEG-IFNa). The amount of interferon may also vary between 10 µg and 300 µg and be for example 10 µg, 30 µg, 50 µg, 70 µg, 90 µg, 100 µg, 120 µg, 130 µg, 140 µg, 150 µg, 160 µg, 170 µg, 180 µg, 190 µg, 200 µg, 210 µg, 220 µg, 230 µg, 240 µg, 250 µg, 260 µg, 270 µg, 280 µg, 290 µg, or 300 µg. The pre-S1 peptide inhibitor and interferon may be administered concurrently. The duration of administration may be 24 weeks or 48 weeks or longer, depending on the progression of the treatment and treatment results.
2. 5 mg pre-S1 peptide inhibitor such as Myrcludex B and 180 µg interferon such as pegylated interferon alpha (PEG-IFNa). The amount of interferon may also vary between 10 µg and 300 µg and be for example 10 µg, 30 µg, 50 µg, 70 µg, 90 µg, 100 µg, 120 µg, 130 µg, 140 µg, 150 µg, 160 µg, 170 µg, 180 µg, 190 µg, 200 µg, 210 µg, 220 µg, 230 µg, 240 µg, 250 µg, 260 µg, 270 µg, 280 µg, 290 µg, or 300 µg. The duration of administration may be 24 weeks or 48 weeks or longer, depending on the progression of the treatment and treatment results.
3. 10 mg pre-S1 peptide inhibitor such as Myrcludex B and 180 µg interferon such as pegylated interferon alpha (PEG-IFNa). The amount of interferon may also vary between 10 µg and 300 µg and be for example 10 µg, 30 µg, 50 µg, 70 µg, 90 µg, 100 µg, 120 µg, 130 µg, 140 µg, 150 µg, 160 µg, 170 µg, 180 µg, 190 µg, 200 µg, 210 µg, 220 µg, 230 µg, 240 µg, 250 µg, 260 µg, 270 µg, 280 µg, 290 µg, or 300 µg. The duration of administration may be 24 weeks or 48 weeks or longer, depending on the progression of the treatment and treatment results.
4. 2 mg pre-S1 peptide inhibitor such as Myrcludex B and 100 mg lamivudine. The amount of lamivudine may also vary between 10 and 100 mg, and be for example 10 mg, 20 mg, 30 mg, 40 mg, 50 mg, 60 mg, 70 mg, 80 mg, 90 mg or 100 mg. The duration of administration may be 24 weeks or 48 weeks or longer, depending on the progression of the treatment and treatment results.
5. 5 mg pre-S1 peptide inhibitor such as Myrcludex B and 100 mg lamivudine. The amount of lamivudine may also vary between 10 and 100 mg, and be for example 10 mg, 20 mg, 30 mg, 40 mg, 50 mg, 60 mg, 70 mg, 80 mg, 90 mg or 100 mg. The duration of administration may be 24 weeks or 48 weeks or longer, depending on the progression of the treatment and treatment results.
6. 10 mg pre-S1 peptide inhibitor such as Myrcludex B and 100 mg lamivudine. The amount of lamivudine may also vary between 10 and 100 mg, and be for example 10 mg, 20 mg, 30 mg, 40 mg, 50 mg, 60 mg, 70 mg, 80 mg, 90 mg or 100 mg. The duration of administration may be 24 weeks or 48 weeks or longer, depending on the progression of the treatment and treatment results.
7. 2 mg pre-S1 peptide inhibitor such as Myrcludex B and 0.5 mg or 1.0 mg entecavir. The amount of entecavir may also vary between 0.1 and 10 mg and be for example 0.1 mg, 0.2 mg, 0.3 mg, 0.4 mg, 0.5 mg, 0.6 mg, 0.7 mg, 0.8 mg, 0.9 mg, 1.0 mg, 2.0 mg, 3.0 mg, 4.0 mg, 5.0 mg, 6.0 mg. 7.0 mg, 8.0 mg, 9.0 mg, or 10.0 mg. The duration of administration may be 24 weeks or 48 weeks or longer, depending on the progression of the treatment and treatment results.
8. 5 mg pre-S1 peptide inhibitor such as Myrcludex B and 0.5 mg or 1.0 mg entecavir. The amount of entecavir may also vary between 0.1 and 10 mg and be for example 0.1 mg, 0.2 mg, 0.3 mg, 0.4 mg, 0.5 mg, 0.6 mg, 0.7 mg, 0.8 mg, 0.9 mg, 1.0 mg, 2.0 mg, 3.0 mg, 4.0 mg, 5.0 mg, 6.0 mg. 7.0 mg, 8.0 mg, 9.0 mg, or 10.0 mg. The duration of administration may be 24 weeks or 48 weeks or longer, depending on the progression of the treatment and treatment results.
9. 10 mg pre-S1 peptide inhibitor such as Myrcludex B and 0.5 mg or 1.0 mg entecavir. The amount of entecavir may also vary between 0.1 and 10 mg and be for example 0.1 mg, 0.2 mg, 0.3 mg, 0.4 mg, 0.5 mg, 0.6 mg, 0.7 mg, 0.8 mg, 0.9 mg, 1.0 mg, 2.0 mg, 3.0 mg, 4.0 mg, 5.0 mg, 6.0 mg. 7.0 mg, 8.0 mg, 9.0 mg, or 10.0 mg. The duration of administration may be 24 weeks or 48 weeks or longer, depending on the progression of the treatment and treatment results.
10. 2 mg pre-S1 peptide inhibitor such as Myrcludex B and 600 mg telbivudine. The amount of telbivudine may also vary between 100 and 1000 mg, and be for example 100 mg, 200 mg, 300 mg, 400 mg, 500 mg, 600 mg, 700 mg, 800 mg, 900 mg, or 1000 mg. The duration of administration may be 24 weeks or 48 weeks or longer, depending on the progression of the treatment and treatment results.
11. 5 mg pre-S1 peptide inhibitor such as Myrcludex B and 600 mg telbivudine. The amount of telbivudine may also vary between 100 and 1000 mg, and be for example 100 mg, 200 mg, 300 mg, 400 mg, 500 mg, 600 mg, 700 mg, 800 mg, 900 mg, or 1000 mg. The duration of administration may be 24 weeks or 48 weeks or longer, depending on the progression of the treatment and treatment results.
12. 10 mg pre-S1 peptide inhibitor such as Myrcludex B and 600 mg telbivudine. The amount of telbivudine may also vary between 100 and 1000 mg, and be for example 100 mg, 200 mg, 300 mg, 400 mg, 500 mg, 600 mg, 700 mg, 800 mg, 900 mg, or 1000 mg. The duration of administration may be 24 weeks or 48 weeks or longer, depending on the progression of the treatment and treatment results.
13. 2 mg pre-S1 peptide inhibitor such as Myrcludex B and 200 mg, 245 mg, or 300 mg tenofovir. The amount of tenofovir may also vary between 100 and 1000 mg, and be for example 100 mg, 200 mg, 300 mg, 400 mg, 500 mg, 600 mg, 700 mg, 800 mg, 900 mg, or 1000 mg. The duration of administration may be 24 weeks or 48 weeks or longer, depending on the progression of the treatment and treatment results.
14. 5 mg pre-S1 peptide inhibitor such as Myrcludex B and 200 mg, 245 mg, or 300 mg tenofovir. The amount of tenofovir may also vary between 100 and 1000 mg, and be for example 100 mg, 200 mg, 300 mg, 400 mg, 500 mg, 600 mg, 700 mg, 800 mg, 900 mg, or 1000 mg. The duration of administration may be 24 weeks or 48 weeks or longer, depending on the progression of the treatment and treatment results.
15. 10 mg pre-S1 peptide inhibitor such as Myrcludex B and 200 mg, 245 mg, or 300 mg tenofovir. The amount of tenofovir may also vary between 100 and 1000 mg, and be for example 100 mg, 200 mg, 300 mg, 400 mg, 500 mg, 600 mg, 700 mg, 800 mg, 900 mg, or 1000 mg. The duration of administration may be 24 weeks or 48 weeks or longer, depending on the progression of the treatment and treatment results.
16. 2 mg pre-S1 peptide inhibitor such as Myrcludex B and 100 mg adefovir. The amount of adefovir may also vary between 5 to 20 mg, and be for example 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg. The duration of administration may be 24 weeks or 48 weeks or longer, depending on the progression of the treatment and treatment results.
17. 5 mg pre-S1 peptide inhibitor such as Myrcludex B and 100 mg adefovir. The amount of adefovir may also vary between 5 to 20 mg, and be for example 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg. The duration of administration may be 24 weeks or 48 weeks or longer, depending on the progression of the treatment and treatment results.
18. 10 mg pre-S1 peptide inhibitor such as Myrcludex B and 100 mg adefovir. The amount of adefovir may also vary between 5 to 20 mg, and be for example 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg. The duration of administration may be 24 weeks or 48 weeks or longer, depending on the progression of the treatment and treatment results.

The duration of administration may be 24 weeks or 48 weeks or longer, depending on the progression of the treatment and side effects.

An inhibitor and a further active ingredient may be administered sequentially. For example, an inhibitor may be administered for at least one cycle or a course comprising one or more cycles (such as 2, 3, 4, 5, 6, 7, 8, 9 or 10 cycles) followed by the administration of a further active ingredient for at least one cycle or a course comprising one or more cycles (such as 2, 3, 4, 5, 6, 7, 8, 9 or 10 cycles). The duration of one cycle may be 1, 2, 3, 4, 5, 6, 7, 8, 9, 10, 11, or 12 weeks. The duration of one cycle may be at least 1, 2, 3, 4, 5, 6, 7, 8, 9, 10, 11, or 12 weeks. The duration of one course may be 12 weeks, 24 weeks, 36 weeks, 48 weeks, 60 weeks, 1 year, 1.1 years, 1.2 years, 1.3 years, 1.4 years, 1.5 years, 1.6 years, 1.7 years, 1.8 years, 1.8 years, 1.9 years, or 2.0 years, or 3 years, or 4 years or longer.

An inhibitor and a further active ingredient may be administered concomitantly/concurrently. According to this administration schedule, the administration of an inhibitor timely overlaps with the administration of a further active ingredient. The duration of the administration of an inhibitor and a further active ingredient may be identical or essentially identical. For example, both an inhibitor and a further active ingredient may be administered for at least one cycle or a course comprising one or more cycles (such as 2, 3, 4, 5, 6, 7, 8, 9 or 10 cycles). The duration of one cycle may be 1, 2, 3, 4, 5, 6, 7, 8, 9, 10, 11, or 12 weeks. The duration of one cycle may be at least 1, 2, 3, 4, 5, 6, 7, 8, 9, 10, 11, or 12 weeks. The duration of one course may be 12 weeks, 24 weeks, 36 weeks, 48 weeks, 60 weeks, 1 year, 1.1 years, 1.2 years, 1.3 years, 1.4 years, 1.5 years, 1.6 years, 1.7 years, 1.8 years, 1.8 years, 1.9 years, or 2.0 years, or 3 years, or 4 years. For example, a 24 weeks course of Myrcludes B may be administered at the same time as a 24 weeks course of PEG-IFNa. In this administration scheme, Myrcludex B may be administered daily, whereas PEG-IFNa may be administered weekly. In this administration schedule, an inhibitor and a further active ingredient may be administered simultaneously, for example at essentially the same time or in a single composition.

An inhibitor and a further active ingredient maybe delivered by various delivery routes, depending on the type of ingredient. Administration routs include enteral route (e.g., orally and rectally), parenteral route (e.g. intravenously, intramuscularly, subcutaneously intraperitoneally) and topically. Preferably, pre-S1 peptide inhibitor is delivered subcutaneously. Preferably, PEG-IFNa is delivered subcutaneously.

Various combinations of active ingredients as described above administered at various doses, treatment schedules and routes of administration as described above. For example, provided herein are the following treatment regimens:
2 mg Myrcludes B given daily for 24 weeks in combination with 180 µg pegylated interferon alpha (PEG-IFNa) given once a week. The course may be followed by the administration of 2 mg daily Myrcludex B alone for another 24 weeks. Both active ingredients may be delivered subcutaneously. In this example, the daily Myrcludex B dose may also be 5 mg or 10 mg.

2 mg Myrcludes B given daily in combination with a weekly dose of 180 µg pegylated interferon alpha (PEG-IFNa) for. The treatment may last 48 weeks. In this example, the daily Myrcludex B dose may also be 5 mg or 10 mg.

2 mg Myrcludes B given daily in combination with 245 mg tenofovir given daily. The treatment may last for 2 years or longer (indefinitely). In this example, the daily Myrcludex B dose may also be 5 mg or 10 mg.

2 mg Myrcludes B may given daily in combination with 245 mg tenofovir given daily. The treatment may last at least 48 weeks. In this example, the daily Myrcludex B dose may be 5 mg or 10 mg.

2 mg Myrcludes B given daily in combination with 1.0 mg entecavir given daily. The treatment may last for 2 years or longer (indefinitely). In this example, the daily Myrcludex B dose may also be 5 mg or 10 mg.

2 mg Myrcludes B may given daily in combination with 1.0 mg entecavir given daily. The treatment may last at least 48 weeks. In this example, the daily Myrcludex B dose may be 5 mg or 10 mg.

The dose of each active ingredient may be adjusted according to the treatment progression and/or side effects the patient develops during the treatment.

### EXAMPLES

### Example 1: Phase 2 clinical trial on Mycludex B in chronic HBV infection

### Objective:

The objective of the clinical trial is the evaluation of safety and tolerability, as well as antiviral efficacy of Myrcludex B in HBsAg-positive patients with active hepatitis.

### Methodology:

Cohort A: 40 chronically HBV infected, HBeAg negative patients (all HBV DNA >2000 IU/ml median HBV DNA 4.7 logic₁₀ IU/ml; no cirrhosis) were treated for 12 weeks with once daily sc 0.5mg, 1mg, 2mg, 5mg or 10mg Myrcludex B for 12 weeks (8 patients per dose). Treatment was extended to 24 weeks in patients receiving 10mg.

### Results:

Myrcludex B was very well tolerated, injection side dermatitis occurred in 3 patients receiving 10mg of Myrcludex B, regressed on treatment. Over 1log₁₀ HBV DNA decline at week 12 was observed in 6/8 (75%) patients receiving 10mg Myrcludex B while this occurred less often in the remaining dose groups (7/40; 17%). Alanine transaminase (ALT) normalized in 22/40 (55%) patients and median ALT values declined from 76 U/l before therapy to 36 U/l at week 12 (p<0.001). No significant changes in HBsAg levels became evident.

### Conclusion:

Myrcludex B is a drug candidate for the treatment of chronic hepatitis B (CHB) and chronic hepatitis delta (CHD). Myrcludex B was well tolerated. However, Myrcludex B did not influence the HBsAg levels.

### Reference Example 2: In vitro study of a combination of Myrcludex B and entecavir in HBV/HDV infected cells

A nucleoside analogue entecavir did not improve the inhibition of HBV receptor by Myrcludex B *in vitro* (measured by immunochemistry evaluation of number of HepaRG cells infected with HBV/HDV containing serum):

**Table 1: Number of HBcAg-positive cells on day 6 post infection as determined by HBcAgspecific IF after infection of HepaRG cells with an HDV-positive serum (serum 1) using increasing concentrations of Myrcludex B in the absence of entecavir and in the presence of 20 µM entecavir.**

| Myrcludex B concentration (nM) | No entecavir | 20 µM entecavir |
|---|---|---|
| | Mean value (% uncomp. infection) | Mean value (% uncomp. infection) |
| 0 | 100 | 100 |
| 0.025 | 44.7 | 36.7 |
| 0.625 | 16.5 | 28.1 |
| 10 | 0.2 | 0.2 |
| 100 | 0 | 0.2 |

Similar results are expected for a combination of Myrcludex B with any nucleoside and nucleotide analogue because they all essentially have the same mechanism of action, i.e., inhibition of HBV polymerase.

### Example 3: In vitro study of a combination of Myrcludex B and IFNa in HBV infected cells

Cell cultures: primary human hepatocytes (PHH), HBV-susceptible cell line HepaRG, cell lines transfected or transduced with HBV receptor NTCP.

Cell cultures will be infected with HBV. Addition of different concentrations of Myrcludex and interferon will be investigated in order to "cure" the cell culture from infection.

### Example 4: Pilot clinical trial with combination therapy of Mycludex B and pegylated IFNa in chronic HDV infection

Myrcludex B did not demonstrate an impact on HBsAg levels *in vivo* (clinical trials with Myrcludex B as monotherapy). The effect of interferon on HBsAg loss in clinical praxis is very modest, with about 3% of patients losing HBsAg after standard 48 weeks therapy. Therefore, it is not obvious that interferon would enhance the impact of Myrcludex B on HBV or HDV infection in terms of reducing HBsAg levels and finally achieving HBsAg loss, i.e., the ultimate goal of HBV therapy.

### Objective:

The objective of the clinical trial is the evaluation of safety and tolerability, as well as antiviral efficacy of Myrcludex B in combination with pegylated interferon alpha (PEG-IFNa) in hepatitis D patients. In particular, effects on virological parameters including HBsAg decline and loss are investigated.

### Methodology:

Cohort B: 24 patients with hepatitis D (compensated liver disease; 12.5% cirrhosis).

Group B1 ("Myrcludex B monotherapy"): 8 patients scheduled for 24 weeks therapy of 2 mg Myrcludex B daily, delivered subcutaneously.

Group B2: ("PEG-IFNa monotherapy"): 8 patients scheduled for 24 weeks therapy of 180 µg pegylated interferon alpha (PEG-IFNa) weekly, delivered subcutaneously.

Group B3 ("combination therapy"): 8 patients scheduled for 24 weeks therapy of 2 mg Myrcludex B daily, delivered subcutaneously in combination with 180 µg pegylated interferon alpha (PEG-IFNa) weekly, delivered subcutaneously.

Pre-treatment with Myrcludex B before start of interferon treatment, and/or post-treatment with Myrcludex B after end of interferon treatment will also be considered.

### Results:

Myrcludex B was very well tolerated. A psoriasis exacerbation occurred in one HDV patient (B3) leading to discontinuation. In groups B1 and B3 each, one patient discontinued the treatment for various reasons.

Six of the seven patients experienced >1log10 HDV RNA decline at week 24 during Myrcludex B monotherapy (group B1, Myrcludex B monotherapy) while this response was observed in 7/7 patients of group B2 (PEG-IFNa monotherapy) and group B3 (combination therapy).

HDV RNA became negative in 2/7 patients of groups B1 (Myrcludex B monotherapy) and B2 (PEG-IFNa monotherapy). In contrast, HDV RNA became negative in 5/7 patients (71%) of group B3 (combination therapy).

ALT values declined at week 24 in 6/7 patients (group B1) and 4/7 patients (group B3) and at week 12 in 3/7 patients (group B2,).

One patient (group B3) and 3 patients (group B2) experienced >0.5 log10 HBsAg decline at week 24.

### Conclusion:

Myrcludex B is safe and well tolerated in HBsAg positive patients with or without HDV co-infection. HBV entry inhibition is associated with HBV DNA and HDV RNA declines and improvement of biochemical disease activity. The effect is potentiated by the Myrcludex B/PEG-IFNa combination therapy, wherein complete HDV RNA eradication was observed in over 70% of patients. The reduction in serum HDV RNA is an indication for the reduction of the number of the virus-producing cells when treated with an pre-S1 cell entry inhibitor, without any direct effect on viral replication. In this study, a synergistic effect of pre-S1 peptide inhibitor with interferon was shown on the HDV RNA levels. This effect will translate into a long term viral suppression, also for HBV, and, eventually, lead to viral eradication. Thus, the observed HDV RNA decline and hence virus eradication can be taken as a marker of effective hepatitis D treatment which is expected to project in HBsAg decline and effective hepatitis B treatment with extended treatment duration..

### Example 5: Phase 2 clinical trial with combination therapy of Mycludex B and pegylated IFNa in chronic HBV infection

### Objective:

The objective of the clinical trial is the evaluation of safety and tolerability, as well as antiviral efficacy of Myrcludex B in combination with pegylated interferon alpha (PEG-IFNa) in in patients with chronic HBV infection. In particular, effects on virological parameters including HBsAg decline and loss are investigated.

### Methodology:

Group 1: Pegylated interferon
Group 2: Pegylated interferon with 2 mg Myrcludex B
Group 3: Pegylated interferon in combination with 5 mg Myrcludex B
Group 4: Pegylated interferon in combination with 10 mg Myrcludex B

The treatment with a combination of Myrcludex B and pegylated interferon will be pursued for 24 weeks, followed by 24 week of Myrcludex B monotherapy. The combination will allow increase of the interferon efficacy and the reduction of treatment duration (48 weeks are standard in the treatment with interferons).

### Reference Example 6: Phase 2 clinical trial with combination therapy of Mycludex B and nucleoside/nucleotide analogues in chronic HBV infection

Myrcludex B did not influence HbsAg levels *in vivo* (clinical trials with Myrcludex B as monotherapy). Treatment with nucleoside/nucleotide analogues resulted in HBsAg seroconversion in a very minor proportion of patients (4% of tenofovir patients after two years of treatment and is even lower for the other drugs). Therefore, it is not obvious that nucleoside/nucleotide analogues would enhance the impact of Myrcludex B on HBV or HDV infection in terms of reducing HBsAg levels and finally achieving HBsAg loss, i.e., the ultimate goal of HBV therapy.

### Objective:

The objective of the clinical trial is the evaluation of safety and tolerability, as well as antiviral efficacy of Myrcludex B in combination with a nucleoside analogue (entecavir) or a nucleotide analogue (tenofovir) in patients with chronic HBV infection. In particular, effects on virological parameters including HBsAg decline and loss are investigated.

### Methodology:

Group 1: Nucleoside or nucleotide analogue
Group 2: Nucleoside or nucleotide analogue in combination with 2 mg Myrcludex B
Group 3: Nucleoside or nucleotide analogue in combination with 5 mg Myrcludex B
Group 4: Nucleoside or nucleotide analogue in combination with 10 mg Myrcludex B

The administration of the active ingredients in the above combinations is concurrent.

Separate trials might be performed in treatment-naive patients, and in patients already receiving nucleoside or nucleotide analogue.

Pre-treatment with Myrcludex B before start of nucleoside or nucleotide analogue treatment, and/or post-treatment with Myrcludex B after end of nucleoside or nucleotide analogue treatment will be considered.

### Example 7: Phase 2 clinical trial with combination therapy of Mycludex B and pegylated IFNa in chronic HDV infection

### Objective:

The objective of the clinical trial is the evaluation of safety and tolerability, as well as antiviral efficacy of Myrcludex B in combination with pegylated interferon alpha (PEG-IFNa) in in patients with chronic HDV infection. In particular, effects on virological parameters including HDV RNA loss, HBsAg decline and loss are investigated.

### Methodology:

Group 1: Pegylated interferon
Group 2: Pegylated interferon with 2mg Myrcludex B
Group 3: Pegylated interferon in combination with 5mg Myrcludex B
Group 4: Pegylated interferon in combination with 10mg Myrcludex B

The treatment with a combination of Myrcludex B and pegylated interferon will be pursued for 24 weeks, followed by 24 week of Myrcludex B monotherapy. The combination will allow increase of the interferon efficacy and the reduction of treatment duration (48 weeks are standard in treatment with interferons).

## Claims

1. A composition or combination comprising as a first constituent an inhibitor of Na+-taurocholate cotransporting polypeptide (NTCP) and as a second constituent an immunomodulator for use in a method of treatment of HBV or HDV infection, hepatitis B, chronic hepatitis B, hepatitis D, or chronic hepatitis D in a subject, wherein the immunomodulator is a pegylated interferon alpha, and wherein the NTCP inhibitor is a pre-S1 peptide inhibitor, wherein the pre-S1 peptide inhibitor comprises a pre-S1 peptide of an HBV virus, and wherein a unit dose of pre-S1 peptide inhibitor is 2 mg, 5 mg, or 10 mg and a unit dose of interferon is 180 µg; wherein the pre-S1 peptide inhibitor is N-Myristoyl-glycyl-L-threonyl-L-asparaginyl-L-leucyl-L-seryl-L-valyl-L-prolyl-L-asparaginyl-L-prolyl-L-leucyl-glycyl-L-phenylalanyl-L-phenylalanyl-L-prolyl-L-aspartyl-L-histidyl-L-glutaminyl-L-leucyl-L-aspartyl-L-prolyl-L-alanyl-L-phenylalanyl-glycyl-L-alanyl-L-asparaginyl-L-seryl-L-asparaginyl-L-asparaginyl-L-prolyl-L-aspartyl-L-tryptophanyl-L-aspartyl-L-phenylalanyl-L-asparaginyl-L-prolyl-L-asparaginyl-L-lysyl-L-aspartyl-L-histidyl-L-tryptophanyl-L-prolyl-L-glutamyl-L-alanyl-L-asparaginyl-L-lysyl-L-valyl-glycinamide, acetate salt.

2. The composition or combination for use of claim 1, wherein the interferon is pegylated interferon alpha 2a or pegylated interferon alpha 2b.

3. The composition or combination for use of claims 1-2, wherein the method comprises administering the NTCP inhibitor and/or the interferon for 12 weeks, 24 weeks, 36 weeks, 48 weeks, 60 weeks, 1 year, 1.1 years, 1.2 years, 1.3 years, 1.4 years, 1.5 years, 1.6 years, 1.7 years, 1.8 years, 1.8 years, 1.9 years, or 2.0 years, or 3 years, or 4 years or longer.

4. The composition or combination for use of claims 1-3, wherein the method comprises administering the NTCP inhibitor and/or the interferon parenterally, preferably intravenously or subcutaneously.

5. The composition or combination for use of claims 1-4, wherein the subject is a human.

## Patentansprüche

1. Zusammensetzung oder Kombination, die als ersten Bestandteil einen Inhibitor von NTCP (Na⁺-Taurocholate Cotransporting Polypeptide) und als zweiten Bestandteil einen Immunmodulator umfasst, zur Verwendung bei einem Verfahren zur Behandlung von HBV- oder HDV-Infektion, Hepatitis B, chronischer Hepatitis B, Hepatitis D oder chronischer Hepatitis D bei einem Individuum, wobei es sich bei dem Immunmodulator um ein pegyliertes Interferon alpha handelt und wobei es sich bei dem NTCP-Inhibitor um einen Prä-S1-Peptid-Inhibitor handelt, wobei der Prä-S1-Peptid-Inhibitor ein Prä-S1-Peptid und ein HBV-Virus umfasst und wobei eine Einzeldosis des Prä-S1-Peptid-Inhibitors 2 mg, 5 mg oder 10 mg beträgt und wobei eine Einzeldosis von Interferon 180 µg beträgt; wobei es sich bei dem Prä-S1-Peptid-Inhibitor um N-Myristoyl-glycyl-L-threonyl-L-asparaginyl-L-leucyl-L-seryl-L-valyl-L-prolyl-L-asparaginyl-L-prolyl-L-leucyl-glycyl-L-phenylalanyl-L-phenylalanyl-L-prolyl-L-aspartyl-L-histidyl-L-glutaminyl-L-leucyl-L-aspartyl-L-prolyl-L-alanyl-L-phenylalanyl-glycyl-L-alanyl-L-asparaginyl-L-seryl-L-asparaginyl-L-asparaginyl-L-prolyl-L-aspartyl-L-tryptophanyl-L-aspartyl-L-phenylalanyl-L-asparaginyl-L-prolyl-L-asparaginyl-L-lysyl-L-aspartyl-L-histidyl-L-tryptophanyl-L-prolyl-L-glutamyl-L-alanyl-L-asparaginyl-L-lysyl-L-valyl-glycinamid-Acetatsalz handelt.

2. Zusammensetzung oder Kombination zur Verwendung nach Anspruch 1, wobei es sich bei dem Interferon um pegyliertes Interferon alpha 2a oder pegyliertes Interferon alpha 2b handelt.

3. Zusammensetzung oder Kombination zur Verwendung nach den Ansprüchen 1-2, wobei das Verfahren das Verabreichen des NTCP-Inhibitors und/oder des Interferons über einen Zeitraum von 12 Wochen, 24 Wochen, 36 Wochen, 48 Wochen, 60 Wochen, 1 Jahr, 1,1 Jahre, 1,2 Jahre, 1,3 Jahre, 1,4 Jahre, 1,5 Jahre, 1,6 Jahre, 1,7 Jahre, 1,8 Jahre, 1,8 Jahre, 1,9 Jahre oder 2,0 Jahre oder 3 Jahre oder 4 Jahre oder länger umfasst.

4. Zusammensetzung oder Kombination zur Verwendung nach den Ansprüchen 1-3, wobei das Verfahren das parenterale, vorzugsweise intravenöse oder subkutane, Verabreichen des NTCP-Inhibitors und/oder des Interferons umfasst.

5. Zusammensetzung oder Kombination zur Verwendung nach den Ansprüchen 1-4, wobei es sich bei dem Individuum um einen Menschen handelt.

## Revendications

1. Composition ou combinaison comprenant en tant que premier constituant un inhibiteur de polypeptide de cotransport de Na+-taurocholate (NTCP) et en tant que deuxième constituant un immunomodulateur pour une utilisation dans un procédé de traitement d'une infection par le VHB ou le VHD, de l'hépatite B, de l'hépatite B chronique, de l'hépatite D ou de l'hépatite D chronique chez un sujet, 1'immunomodulateur étant un interféron alpha pegylé et l'inhibiteur de NTCP étant un inhibiteur de peptide pré-S1, l'inhibiteur de peptide pré-S1 comprenant un peptide pré-S1 d'un virus VHB, et une dose unitaire d'inhibiteur de peptide pré-S1 étant de 2 mg, 5 mg ou 10 mg et une dose unitaire d'interféron étant de 180 µg ; l'inhibiteur de peptide pré-S1 étant le sel d'acétate de N-Myristoyl-glycyl-L-thréonyl-L-asparaginyl-L-leucyl-L-séryl-L-valyl-L-prolyl-L-asparaginyl-L-prolyl-L-leucyl-glycyl-L-phénylalanyl-L-phénylalanyl-L-prolyl-L-aspartyl-L-histidyl-L-glutaminyl-L-leucyl-L-aspartyl-L-prolyl-L-alanyl-L-phénylalanyl-glycyl-L-alanyl-L-asparaginyl-L-séryl-L-asparaginyl-L-asparaginyl-L-prolyl-L-aspartyl-L-tryptophanyl-L-aspartyl-L-phénylalanyl-L-asparaginyl-L-prolyl-L-asparaginyl-L-lysyl-L-aspartyl-L-histidyl-L-tryptophanyl-L-prolyl-L-glutamyl-L-alanyl-L-asparaginyl-L-lysyl-L-valyl-glycinamide.

2. Composition ou combinaison pour une utilisation selon la revendication 1, l'interféron étant l'interféron alpha 2a pegylé ou l'interféron alpha 2b pegylé.

3. Composition ou combinaison pour une utilisation selon les revendications 1 à 2, le procédé comprenant l'administration de l'inhibiteur de NTCP et/ou de l'interféron pendant 12 semaines, 24 semaines, 36 semaines, 48 semaines, 60 semaines, 1 an, 1,1 an, 1,2 an, 1,3 an, 1,4 an, 1,5 ans, 1,6 ans, 1,7 ans, 1,8 ans, 1,8 ans, 1,9 ans ou 2,0 ans, ou 3 ans, ou 4 ans ou plus longtemps.

4. Composition ou combinaison pour une utilisation selon les revendications 1 à 3, le procédé comprenant l'administration de l'inhibiteur de NTCP et/ou de l'interféron de manière parentérale, préférablement de manière intraveineuse ou de manière sous-cutanée.

5. Composition ou combinaison pour une utilisation selon les revendications 1 à 4, le sujet étant un humain.


### SEQUENCE LISTING

<110> MYR GmbH (Burgwedel)
<120> COMBINATION THERAPY OF HBV AND HDV INFECTION
<130> 103648PC
<150> EP14187865.2
   <151> 2014-10-07
<160> 14
<170> PatentIn version 3.5
<210> 1
   <211> 59
   <212> PRT
   <213> artificial
<220>
   <223> HBV preS1 consensus sequence (positions (-11) to 48)
<400> 1
   Met Gly Gly Trp Ser Ser Thr Pro Arg Lys Gly Met Gly Thr Asn Leu
   1               5                   10                  15
   Ser Val Pro Asn Pro Leu Gly Phe Phe Pro Asp His Gln Leu Asp Pro
               20                  25                  30
   Ala Phe Arg Ala Asn Ser Asn Asn Pro Asp Trp Asp Phe Asn Pro Asn
           35                  40                  45
   Lys Asp His Trp Pro Glu Ala Asn Lys Val Gly
       50                  55
<210> 2
   <211> 59
   <212> **PRT**
   <213> Hepatitis B virus
<400> 2
   Met Gly Gly Trp Ser Ser Lys Pro Arg Lys Gly Met Gly Thr Asn Leu
   1               5                   10                  15
   Ser Val Pro Asn Pro Leu Gly Phe Phe Pro Asp His Gln Leu Asp Pro
               20                  25                  30
   Ala Phe Gly Ala Asn Ser Asn Asn Pro Asp Trp Asp Phe Asn Pro Val
           35                  40                  45
   Lys Asp Asp Trp Pro Ala Ala Asn Gln Val Gly
       50                  55
<210> 3
   <211> 59
   <212> **PRT**
   <213> Hepatitis B virus
<400> 3
   Met Gly Gly Trp Ser Ser Lys Pro Arg Lys Gly Met Gly Thr Asn Leu
   1               5                   10                  15
   Ser Val Pro Asn Pro Leu Gly Phe Phe Pro Asp His Gln Leu Asp Pro
               20                  25                  30
   Ala Phe Lys Ala Asn Ser Glu Asn Pro Asp Trp Asp Leu Asn Pro His
           35                  40                  45
   Lys Asp Asn Trp Pro Asp Ala Asn Lys Val Gly
       50                  55
<210> 4
   <211> 59
   <212> **PRT**
   <213> Hepatitis B virus
<400> 4
   Met Gly Gly Trp Ser Ser Lys Pro Arg Gln Gly Met Gly Thr Asn Leu
   1               5                   10                  15
   Ser Val Pro Asn Pro Leu Gly Phe Phe Pro Asp His Gln Leu Asp Pro
               20                  25                  30
   Ala Phe Gly Ala Asn Ser Asn Asn Pro Asp Trp Asp Phe Asn Pro Asn
           35                  40                  45
   Lys Asp His Trp Pro Glu Ala Asn Gln Val Gly
       50                  55
<210> 5
   <211> 48
   <212> **PRT**
   <213> Hepatitis B virus
<400> 5
   Met Gly Gln Asn Leu Ser Thr Ser Asn Pro Leu Gly Phe Phe Pro Asp
   1               5                   10                  15
   His Gln Leu Asp Pro Ala Phe Arg Ala Asn Thr Ala Asn Pro Asp Trp
               20                  25                  30
   Asp Phe Asn Pro Asn Lys Asp Thr Trp Pro Asp Ala Asn Lys Val Gly
             35                  40                  45
<210> 6
   <211> 58
   <212> PRT
   <213> Hepatitis B virus
<400> 6
   Met Gly Leu Ser Trp Thr Val Pro Leu Glu Trp Gly Lys Asn Ile Ser
   1               5                   10                  15
   Thr Thr Asn Pro Leu Gly Phe Phe Pro Asp His Gln Leu Asp Pro Ala
               20                  25                  30
   Phe Arg Ala Asn Thr Arg Asn Pro Asp Trp Asp His Asn Pro Asn Lys
           35                  40                  45
   Asp His Trp Thr Glu Ala Asn Lys Val Gly
       50                  55
<210> 7
   <211> 59
   <212> **PRT**
   <213> Hepatitis B virus
<400> 7
   Met Gly Ala Pro Leu Ser Thr Thr Arg Arg Gly Met Gly Gln Asn Leu
   1               5                   10                  15
   Ser Val Pro Asn Pro Leu Gly Phe Phe Pro Asp His Gln Leu Asp Pro
               20                  25                  30
   Leu Phe Arg Ala Asn Ser Ser Ser Pro Asp Trp Asp Phe Asn Thr Asn
           35                  40                  45
   Lys Asp Ser Trp Pro Met Ala Asn Lys Val Gly
       50                  55
<210> 8
   <211> 58
   <212> PRT
   <213> Hepatitis B virus
<400> 8
   Met Gly Leu Ser Trp Thr Val Pro Leu Glu Trp Gly Lys Asn Leu Ser
   1               5                   10                  15
   Ala Ser Asn Pro Leu Gly Phe Leu Pro Asp His Gln Leu Asp Pro Ala
               20                  25                  30
   Phe Arg Ala Asn Thr Asn Asn Pro Asp Trp Asp Phe Asn Pro Lys Lys
           35                  40                  45
   Asp Pro Trp Pro Glu Ala Asn Lys Val Gly
       50                  55
<210> 9
   <211> 59
   <212> **PRT**
   <213> Hepatitis B virus
<400> 9
   Met Gly Ala Pro Leu Ser Thr Ala Arg Arg Gly Met Gly Gln Asn Leu
   1               5                   10                  15
   Ser Val Pro Asn Pro Leu Gly Phe Phe Pro Asp His Gln Leu Asp Pro
               20                  25                  30
   Leu Phe Arg Ala Asn Ser Ser Ser Pro Asp Trp Asp Phe Asn Thr Asn
           35                  40                  45
   Lys Asp Asn Trp Pro Met Ala Asn Lys Val Gly
       50                  55
<210> 10
   <211> 48
   <212> PRT
   <213> Wooly Monkey Hepatitis B Virus
<400> 10
   Met Gly Leu Asn Gln Ser Thr Phe Asn Pro Leu Gly Phe Phe Pro Ser
   1               5                   10                  15
   His Gln Leu Asp Pro Leu Phe Lys Ala Asn Ala Gly Ser Ala Asp Trp
               20                  25                  30
   Asp Lys Asn Pro Asn Lys Asp Pro Trp Pro Gln Ala His Asp Thr Ala
           35                  40                  45
<210> 11
   <211> 59
   <212> PRT
   <213> Artificial
<220>
   <223> HBV preS1 Genotype C (positions (-11) to 48) with amino acid substitution at position 46 (Gln/Q -> Lys/K)
<400> 11
   Met Gly Gly Trp Ser Ser Lys Pro Arg Gln Gly Met Gly Thr Asn Leu
   1               5                   10                  15
   Ser Val Pro Asn Pro Leu Gly Phe Phe Pro Asp His Gln Leu Asp Pro
               20                  25                  30
   Ala Phe Gly Ala Asn Ser Asn Asn Pro Asp Trp Asp Phe Asn Pro Asn
           35                  40                  45
   Lys Asp His Trp Pro Glu Ala Asn Lys Val Gly
       50                  55
<210> 12
   <211> 47
   <212> PRT
   <213> Artificial
<220>
   <223> HBV preS1 consensus sequence (positions 2 to 48)
<400> 12
   Gly Thr Asn Leu Ser Val Pro Asn Pro Leu Gly Phe Phe Pro Asp His
   1               5                   10                  15
   Gln Leu Asp Pro Ala Phe Arg Ala Asn Ser Asn Asn Pro Asp Trp Asp
               20                  25                  30
   Phe Asn Pro Asn Lys Asp His Trp Pro Glu Ala Asn Lys Val Gly
           35                  40                  45
<210> 13
   <211> 47
   <212> PRT
   <213> Artificial
<220>
   <223> HBV preS1 Genotype C (positions 2 to 48) with amino acid substitution at position 46 (Gln/Q -> Lys/K)
<400> 13
   Gly Thr Asn Leu Ser Val Pro Asn Pro Leu Gly Phe Phe Pro Asp His
   1               5                   10                  15
   Gln Leu Asp Pro Ala Phe Gly Ala Asn Ser Asn Asn Pro Asp Trp Asp
               20                  25                  30
   Phe Asn Pro Asn Lys Asp His Trp Pro Glu Ala Asn Lys Val Gly
           35                  40                  45
<210> 14
   <211> 47
   <212> **PRT**
   <213> Hepatitis B Virus
<400> **14**
   Gly Thr Asn Leu Ser Val Pro Asn Pro Leu Gly Phe Phe Pro Asp His
   1               5                   10                  15
   Gln Leu Asp Pro Ala Phe Gly Ala Asn Ser Asn Asn Pro Asp Trp Asp
               20                  25                  30
   Phe Asn Pro Asn Lys Asp His Trp Pro Glu Ala Asn Gln Val Gly
           35                  40                  45